(19) Europäisches Patentamt
European Patent Office
Office européen des brevets

(11) Veröffentlichungsnummer: **0 121 727**
**B1**

(12) # EUROPÄISCHE PATENTSCHRIFT

(45) Veröffentlichungstag der Patentschrift: **24.09.86**

(21) Anmeldenummer: **84102122.3**

(22) Anmeldetag: **29.02.84**

(51) Int. Cl.⁴: **A 01 N 43/60,** C 07 D 215/56, C 07 D 401/04, C 07 D 401/12, C 07 D 401/14

(54) **Bakterizide Mittel auf Chinoloncarbonsäure-Basis.**

(30) Priorität: **12.03.83 DE 3308909**

(43) Veröffentlichungstag der Anmeldung: **17.10.84 Patentblatt 84/42**

(45) Bekanntmachung des Hinweises auf die Patenterteilung: **24.09.86 Patentblatt 86/39**

(84) Benannte Vertragsstaaten: **AT BE CH DE FR GB IT LI NL**

(56) Entgegenhaltungen: **EP-A-0 049 355**
**FR-A-2 449 682**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Kühle, Engelbert, Dr., von-Bodelschwingh- Strasse 42, D-5060 Bergisch-Gladbach 2 (DE)**
Erfinder: **Kuck, Karl- Heinz, Dr., Heerstrasse 24, D-4018 Langenfeld (DE)**

EP 0 121 727 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft die bakterizide Verwendung von neuen 1-Cyclopropyl-1,4-dihydro-4-oxo-3-chinolincarbonsäure -Derivaten.

Es sind bereits bestimmte 1-Cyclopropyl-1,4-dihydro-4-oxo-3-chinolincarbonsäuren, wie z.B. die 1-Cyclopropyl-6-fluor-1,4-dihydro-4-oxo-7-(1-piperazinyl)-3-chinolincarbonsäure, bekannt (vgl. EP-A2-49 355, EP-A1-84101 442). Über eine mikrobizide Wirkung auf dem Pflanzenschutzgebiet ist nichts bekannt, nur ihre Verwendung auf dem Pharmagebiet.

Weiterhin ist bekannt geworden, daß bestimmte Chinoloncarbonsäurederivate, wie z.B, die 7-Chlor-1-ethyl-6-fluor-1,4-dihydro-4-oxo-3-chinolincarbonsäure, eine bakterizide Wirkung auf dem Pflanzenschutzgebiet aufweisen (vgl. Europäische Patentanmeldung 0203). Die Wirksamkeit ist jedoch bei niedrigen Anwendungskonzentrationen nicht immer befriedigend.

Es wurde die neue Verwendung der 1-Cyclopropyl-1,4-dihydro-4-oxo-3-chinolincarbonsäure-Derivate der Formel (I)

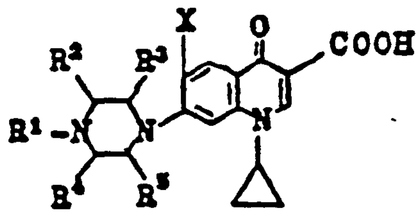

(I)

in der

$R^1$ für einen Rest CO-$R^6$, CN, $SO_2$-$R^7$ oder S-$R^8$ steht,

wobei

$R^6$ für Wasserstoff, gegebenenfalls substituiertes geradkettiges oder verzweigtes Alkyl mit 1 bis 6 Kohlenstoffatomen, gegebenenfalls substituiertes Aryl mit 6 oder 10 Kohlenstoffatomen, gegebenenfalls substituiertes Aralkyl mit 6 Kohlenstoffatomen im Arylteil und 1 bis 3 Kohlenstoffatomen im Alkylteil, gegebenenfalls substituiertes Alkoxy oder Alkylthio mit 1 bis 6 Kohlenstoffatomen, gegebenenfalls substituiertes Phenoxy, gegebenenfalls substituiertes Benzyloxy, Amino, gegebenenfalls substituiertes Alkylamino oder Dialkylamino mit 1 bis 6 Kohlenstoffatomen je Alkylrest oder gegebenenfalls substituiertes Phenylamino steht,

$R^7$ für gegebenenfalls substituiertes geradkettiges oder verzweigtes Alkyl mit 1 bis 5 Kohlenstoffatomen, Phenyl oder Methylphenyl steht,

$R^8$ für Methoxycarbonyl, Trichlormethyl, Trifluormethyl oder Dichlorfluormethyl steht,

$R^2$, $R^3$, $R^4$ und $R^5$ gleich oder verschieden sein können und für Wasserstoff, Methyl, Ethyl, n- oder i-Propyl stehen, und

X für Wasserstoff, Halogen, vorzugsweise Fluor oder Chlor, oder Nitro steht, und deren Säureadditions- Alkali- und Erdalkalisalze, Zink-, Mangan-, und Kupfersalze und Hydrate zur Bekämpfung von Bakterien auf dem Pflanzenschutzgebiet gefunden.

Gegenstand der Empfindung sind auch die Zink-, Mangan-, und Kupfersalze der Formel (I') als solche, wie sie in den Stoffansprüchen definiert sind.

Überraschenderweise zeigen die 1-Cyclopropyl-1,4-dihydro-4-oxo-3-chinolincarbonsäure-Derivate der Formel (I) eine bessere bakterizide Wirkung als die aus dem Stand der Technik bekannte vergleichbare 7-Chlor-1,4-dihydro-1-ethyl-6-fluor-4-oxo-3-chinolincarbonsäure, die wirkungsmäßig die chemisch naheliegendste Verbindung ist. Die neue Verwendung der Wirkstoffe stellt somit eine Bereicherung der Technik dar.

Die erfindungsgemäß verwendbaren Verbindungen sind durch die Formel (I) definiert. In dieser Formel stehen vorzugsweise

$R^1$ für $COR^6$, CN, $SO_2R^7$ oder S-$R^8$;

$R^6$ für Wasserstoff, für gegebenenfalls ein- bis dreifach durch Amino, Chlor, Alkoxycarbonyl mit 1 bis 4 Kohlenstoffatomen im Alkylteil, Carboxy, Alkoxy mit 1 bis 4 Kohlenstoffatomen, Hydroxy, oder Trifluormethylthio substituiertes geradkettiges oder verzweigtes Alkyl mit 1 bis 5 Kohlenstoffatomen, für gegebenenfalls ein- bis fünffach, gleich oder verschieden durch Halogen, Hydroxy, Methoxy, Amino, Nitro oder durch Carboxy substituiertes Phenyl, gegebenenfalls durch Amino im Methylenrest substituiertes Benzyl,

gegebenenfalls durch Fluor, Chlor, Pyrazol-1-yl, 1,2,3-Triazol-1-yl, N-Oxido-2-, -3- oder -4-pyridyl substituiertes Alkoxy oder Alkylthio mit 1 bis 5 Kohlenstoffatomen, für Benzyloxy, Amino, gegebenenfalls durch Alkoxycarbonyl mit 1 bis 3 Kohlenstoffatomen im Alkylteil, Benzyloxycarbonyl, Carboxy oder Halogen substituiertes Alkylamino mit 1 bis 5 Kohlenstoffatomen, Phenylamino,

$R^7$ für gegebenenfalls durch 1 bis 3 Substituenten aus der Reihe Fluor, Chlor oder Amino substituiertes geradketradkettiges oder verzweigtes Alkyl mit 1 bis 4 Kohlenstoffatomen, Phenyl oder Methylphenyl,

$R^8$ für Methoxycarbonyl, Trichlormethyl oder Dichlorfluormethyl,

$R^2$, $R^3$, $R^4$ und $R^5$ für Wasserstoff, Methyl oder Ethyl und

X für Wasserstoff, Fluor, Chlor oder Nitro.

Besonders bevorzugt sind solche Verbindungen der Formel (I), in welcher

$R^1$ für $COR^6$, CN, $SO_2R^7$ oder $S\text{-}R^8$ steht,

$R^6$ für Wasserstoff, gegebenenfalls durch 1 oder 2 Substituenten aus der Reihe Amino, Alkoxycarbonyl mit 1 bis 3 Kohlenstoffatomen im Alkylteil, Carboxy, Alkoxy mit 1 bis 3 Kohlenstoffatomen oder Trifluormethylthio substituiertes geradkettiges oder verzweigtes Alkyl mit 1 bis 4 Kohlenstoffatomen, gegebenenfalls durch 1 bis 5 Substituenten aus der Reihe Chlor, Hydroxy, Amino oder Carboxy substituiertes Phenyl, gegebenenfalls durch Amino im Methylenrest substituiertes Benzyl, gegebenenfalls durch Pyrazol-1-yl, 1,2,3-Triazol-1-yl, N-Oxido-2-, -3- oder -4-pyridyl substituiertes Alkoxy mit 1 bis 4 Kohlenstoffatomen oder Alkylthio mit 1 oder 2 Kohlenstoffatomen, für Benzyloxy, Amino, gegebenenfalls durch Alkoxycarbonyl mit 1 bis 3 Kohlenstoffatomen im Alkylteil, Chlor oder durch Carboxy substituiertes Alkylamino mit 1 bis 5 Kohlenstoffatomen steht,

$R^7$ für geradkettiges oder verzweigtes Alkyl mit 1 bis 3 Kohlenstoffatomen, Dichlorfluormethyl, Chlorbutyl, Phenyl oder Methylphenyl steht,

$R^8$ für Methoxycarbonyl, Trichlormethyl oder Dichlorfluormethyl steht,

$R^2$ für Wasserstoff, Methyl oder Ethyl steht,

$R^3$ für Wasserstoff steht,

$R^4$ für Wasserstoff oder Methyl steht,

$R^5$ für Wasserstoff und

X für Wasserstoff, Fluor, Chlor oder Nitro stehen.

Im einzelnen seien an erfindungsgemäß zu verwendenden Verbindungen der Formel (I) genannt:

7-[4-(2-Carboxy-3,4,5,6-tetrachlorbenzoyl)-1-piperazinyl] -1-cyclopropyl-6-fluor-1,4-dihydro-4-oxo-3-chinolincarbonsäure,

7-[4-(3-Methoxycarbonyl-propionyl-1-piperazinyl]-1-cyclopropyl-6-fluor-1,4-dihydro-4-oxo-3-chinolincarbonsäure,

7-[4-(Trifluormethylthio-acetyl)-1-piperazinyl]-1-cyclopropyl -6-fluor-1,4-dihydro-4-oxo-3-chinolincarbonsäure,

7-[4-Propionyl-1-piperazinyl]-1-cyclopropyl-6-fluor-1,4-dihydro-4-oxo-3-chinolincarbonsäure,

7-[4-Butyryl-1-piperazinyl]-1-cyclopropyl-6-fluor-1,4-dihydro-4-oxo-3-chinolincarbonsäure,

7-[4-(4-Chlorbutyryl)-1-piperazinyl]-1-cyclopropyl-6-fluor-1,4-dihydro-4-oxo-3-chinolincarbonsäure,

7-[4-(3-Methyl-butyryl)-1-piperazinyl]-1-cyclopropyl-6-fluor-1,4-dihydro-4-oxo-3-chinolincarbonsäure,

7-[4-(3-Methoxypropionyl)-1-piperazinyl]-1-cyclopropyl-6-fluor-1,4-dihydro-4-oxo-3-chinolincarbonsäure,

7-[4-Benzoyl-1-piperazinyl]-1-cyclopropyl-6-fluor-1,4-dihydro-4-oxo-3-chinolincarbonsäure,

7-[4-(4-Nitrobenzoyl)-1-piperazinyl]-1-cyclopropyl-6-fluor-1,4-dihydro-4-oxo-3-chinolincarbonsäure,

7-[4-(4-Aminobenzoyl)-1-piperazinyl]-1-cyclopropyl-6-fluor-1,4-dihydro-4-oxo-3-chinolincarbonsäure,

7-[4-(3-Carboxypropionyl)-1-piperazinyl]-1-cyclopropyl-6-fluor-1,4-dihydro-4-oxo-3-chinolincarbonsäure,

7-[4-(4-Carboxybutyryl)-1-piperazinyl]-1-cyclopropyl-6-fluor-1,4-dihydro-4-oxo-3-chinolincarbonsäure,

7-[4-(2-Carboxybenzoyl)-1-piperazinyl]-1-cyclopropyl-6-fluor-1,4-dihydro-4-oxo-3-chinolincarbonsäure,

7-[4-(2-Carboxy-3,4,5,6-tetrachlorbenzoyl]-1-piperazinyl [-1-cyclopropyl-6-fluor-1,4-dihydro-4-oxo-3-chinolincarbonsäure,

7-[4-(3-Methoxycarbonyl-propionyl)-1-piperazinyl]-1-cyclopropyl-6-fluor-1,4-dihydro-4-oxo-3-chinolincarbonsäure,

7-[4-(Hydroxyacetyl)-1-piperazinyl]-1-cyclopropyl-6-fluor-1,4-dihydro-4-oxo-3-chinolincarbonsäure,

7-[4-(Aminoacetyl)-1-piperazinyl]-1-cyclopropyl-6-fluor-1,4-dihydro-4-oxo-3-chinolincarbonsäure,

7-[4-(2-Aminopropionyl)-1-piperazinyl]-1-cyclopropyl-6-fluor-1,4-dihydro-4-oxo-3-chinolincarbonsäure,

7-[4-(2-Amino-3-methyl-butyryl)-1-piperazinyl]-1-cyclopropyl -6-fluor-1,4-dihydro-4-oxo-3-chinolincarbonsäure,

7-[4-(2-Amino-4-methyl-pentanoyl)-1-piperazinyl]-1-cyclopropyl-6-fluor-1,4-dihydro-4-oxo-3-chinolincarbonsäure,

7-[4-(2-Hydroxy-4-amino-butyryl)-1-piperazinyl]-1-cyclopropyl -6-fluor-1,4-dihydro-4-oxo-3-chinolincarbonsäure,

7-[4-Methoxycarbonyl-1-piperazinyl]-1-cyclopropyl-6-fluor-1,4-dihydro-4-oxo-3-chinolincarbonsäure,

7-[4-Ethoxycarbonyl-1-piperazinyl]-1-cyclopropyl-6-fluor-1,4-dihydro-4-oxo-3-chinolincarbonsäure,

7-[4-n-Butoxycarbonyl-1-piperazinyl]-1-cyclopropyl-6-fluor-1,4-dihydro-4-oxo-3-chinolincarbonsäure,

7-[4-tert.-Butoxycarbonyl-1-piperazinyl]-1-cyclopropyl-6-fluor-1,4-dihydro-4-oxo-3-chinolincarbonsäure,

7-{4-[2-(1-Pyrazolyl)-ethyloxycarbonyl]-1-piperazinyl}-1-cyclopropyl-6-fluor-1,4-dihydro-4-oxo-3-chinolincarbonsäure,

7-{4-[2-(1,2,3-Triazol-1-yl)-ethoxycarbonyl]-1-piperazinyl} -1-cyclopropyl-6-fluor-1,4-dihydro-4-oxo-3-chi-

nolincarbonsäure,
7-[4-(2-Chlorethyloxycarbonyl)-1-piperazinyl[-1-cyclopropyl -6-fluor-1,4-dihydro-4-oxo-3-chinolincarbonsäure,
7-{4-[(N-Oxido-3-pyridyl)-methyloxycarbonyl]-1-piperazinyl} -1-cyclopropyl-6-fluor-1,4-dihydro-4-oxo-3-chinolincarbonsäure,
7-[4-Carbamoyl-1-piperazinyl]-1-cyclopropyl-6-fluor-1,4-dihydro-4-oxo-3-chinolincarbonsäure,
7-[4-Methylcarbamoyl-1-piperazinyl]-1-cyclopropyl-6-fluor-1,4-dihydro-4-oxo-3-chinolincarbonsäure,
7-[4-Hexylcarbamoyl-1-piperazinyl]-1-cyclopropyl-6-fluor-1,4-dihydro-4-oxo-3-chinolincarbonsäure,
7-[4-Phenylcarbamoyl-1-piperazinyl[-1-cyclopropyl-6-fluor-1,4-dihydro-4-oxo-3-chinolincarbonsäure,
7-{4-[(3-Methoxycarbonylpropyl)-carbamoyl]-1-piperazinyl}-1-cyclopropyl-6-fluor-1,4-dihydro-4-oxo-3-chinolincarbonsäure,
7-{4-[(5-Methoxycarbonylpentyl)-carbamoyl]-1-piperazinyl }-1-cyclopropyl-6-fluor-1,4-dihydro-4-oxo-3-chinolincarbonsäure,
7-{4-[(3-Carboxypropyl)-carbamoyl]-1-piperazinyl}-1-cyclopropyl-6-fluor-1,4-dihydro-4-oxo-3-chinolincarbonsäure,
7-{4-[(5-Carboxypentyl)-carbamoyl]-1-piperazinyl}-1-cyclopropyl-6-fluor-1,4-dihydro-4-oxo-3-chinolincarbonsäure,
7-{4-[(2-Propoxycarbonyl)-carbamoyl]-1-piperazinyl}-1-cyclopropyl-6-fluor-1,4-dihydro-4-oxo-3-chinolincarbonsäure,
7-{4-[(2-Carboxyethyl)-carbamoyl]-1-piperazinyl}-1 cyclopropyl-6-fluor-1,4-dihydro-4-oxo-3-chinolincarbonsäure,
7-[4-Dimethylcarbamoyl-1-piperazinyl]-1-cyclopropyl-6-fluor-1,4-dihydro-4-oxo-3-chinolincarbonsäure,
7-{4-[(4-Chlorbutyl)-carbamoyl]-1-piperazinyl}-1-cyclopropyl -6-fluor-1,4-dihydro-4-oxo-3-chinolincarbonsäure,
7-[4-Cyano-1-piperazinyl]-1-cyclopropyl-6-fluor-1,4-dihydro -4-oxo-3-chinolincarbonsäure,
7-[4-Trichlormethansulfenyl-1-piperazinyl]-1-cyclopropyl-6-fluor-1,4-dihydro-4-oxo-3-chinolincarbonsäure,
7-[4-Methoxycarbonylsulfenyl-1-piperazinyl]-1-cyclopropyl -6-fluor-1,4-dihydro-4-oxo-3-chinolincarbonsäure,
7-[4-Methansulfonyl-1-piperazinyl]-1-cyclopropyl-6-fluor-1,4-dihydro-4-oxo-3-chinolincarbonsäure,
7-[4-Dichlorfluormethansulfonyl-1-piperazinyl]-1-cyclopropyl -6-fluor-1,4-dihydro-4-oxo-3-chinolincarbonsäure,
7-[4-n-Propansulfonyl-1-piperazinyl]-1-cyclopropyl-6-fluor-1,4-dihydro-4-oxo-3-chinolincarbonsäure,
7-]4-(4-Chlorbutansulfonyl)-1-piperazinyl]-1-cyclopropyl -6-fluor-1,4-dihydro-4-oxo-3-chinolincarbonsäure,
7-[4-Perfluorbutansulfonyl-1-piperazinyl]-1-cyclopropyl -6-fluor-1,4-dihydro-4-oxo-3-chinolincarbonsäure,
7-[4-Acetyl-3-methyl-1-piperazinyl]-1-cyclopropyl-6-fluor-1,4-dihydro-4-oxo-3-chinolincarbonsäure,
7-[3-Methyl-4-propionyl-1-piperazinyl]-1-cyclopropyl-6-fluor-1,4-dihydro-4-oxo-3-chinolincarbonsäure,
7-[3-Methyl-4-methylcarbamoyl-1-piperazinyl]-1-cyclopropyl -6-fluor-1,4-dihydro-4-oxo-3-chinolincarbonsäure,
7-[2,5-Dimethyl-4-formyl-1-piperazinyl]-1-cyclopropyl-6-fluor-1,4-dihydro-4-oxo-3-chinolincarbonsäure,
7-[2,5-Dimethyl-4-butyryl-1-piperazinyl]-1-cyclopropyl -6-fluor-1,4-dihydro-4-oxo-3-chinolincarbonsäure,
7-[3,5-Dimethyl-4-acetyl-1-piperazinyl]-1-cyclopropyl -6-fluor-1,4-dihydro-4-oxo-3-chinolincarbonsäure,
7-[3,5-Dimethyl-4-carbamoyl-1-piperazinyl]-1-cyclopropyl -6-fluor-1,4-dihydro-4-oxo-3-chinolincarbonsäure,
7-(4-Butyryl-1-piperazinyl)-1-cyclopropyl-1,4-dihydro-6-nitro-4-oxo-3-chinolincarbonsäure,
7-(4-Butyryl-1-piperazinyl)-6-chlor-1-cyclopropyl-1,4-dihydro-4-oxo-3-chinolincarbonsäure,
7-(4-Butyryl-1-piperazinyl)-1-cyclopropyl-1,4-dihydro-4-oxo-3-chinolincarbonsäure,
7-[4-(2-Aminopropionyl)-3-methyl-1-piperazinyl]-1-cyclopropyl-6-fluor-1,4-dihydro-4-oxo-3-chinolincarbonsäure,
7-(4-Butyryl-3,5-dimethyl-1-piperazinyl)-1-cyclopropyl -6-fluor-1,4-dihydro-4-oxo-3-chinolincarbonsäure.

Die erfindungsgemäß verwendbaren Verbindungen der Formel (I) können gegebenenfalls mit einer organischen oder anorganischen Säure in ein Salz übergeführt werden. Zur Salzbildung geeignete Säuren sind z.B. Halogenwasserstoffsäuren wie Salzsäure, Bromwasserstoffsäure, Iodwasserstoffsäure, Schwefelsäure, Essigsäure, Zitronensäure, Ascorbinsäure, Methansulfonsäure und Benzolsulfonsäure. Als Alkali- bzw. Erdalkalisalze sind vorzugsweise Natrium-, Kalium-, Calcium- und Magnesiumsalze geeignet, außerdem sind Kupfer-, Zink und Mangansalze geeignet.

Die erfindungsgemäß zu verwendenden Wirkstoffe sind noch nicht bekannt. Sie sind jedoch Gegenstand einer eigenen älteren Patentanmeldung, die noch nicht veröffentlicht ist [vgl. EP-A1- 84 101 442] und werden erhalten, wenn man

a) eine Verbindung der Formel (II)

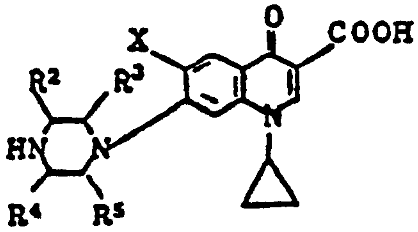

(II),

in welcher
X, $R^2$, $R^3$, $R^4$ und $R^5$ die oben angegebenen Bedeutungen haben, mit einer Verbindung der Formel (III)
$R^1$-Y (III),
in welcher
$R^1$ die oben angegebene Bedeutung hat und
Y für eine Abgangsgruppe wie Halogen, vorzugsweise Chlor, Fluor oder Brom, Methoxy, Ethoxy, Phenoxy, 4-Nitrophenoxy oder 2,3,5-Trichlorphenoxy, Alkoxycarbonyloxy steht, umsetzt oder wenn man
b) Verbindungen der Formel (II)

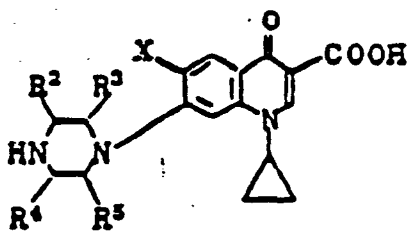

(II)

in welcher
$R^2$, $R^3$ $R^4$, $R^5$ und X die oben angegebene Bedeutung haben, mit Isocyanaten der Formel (IV)
R'-NCO (IV)
in welcher
R' gegebenenfalls substituiertes Alkyl oder Phenyl bedeutet,
zu den erfindungsgemäßen Verbindungen der Formel (Ia)

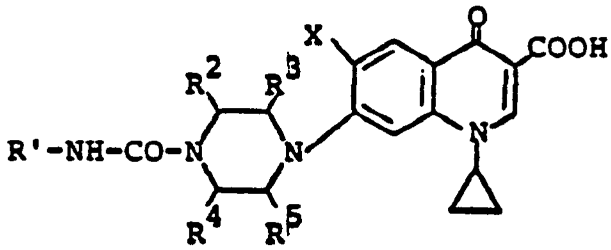

(Ia)

in welcher
R', $R^2$, $R^3$, $R^4$, $R^5$ und X die oben angegebene Bedeutung haben, umsetzt
oder wenn man
c) Verbindungen der Formel (II)

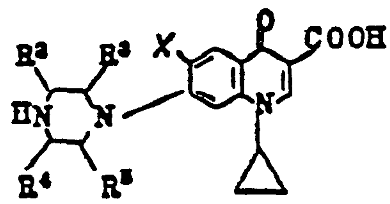

(II)

in welcher
$R^2$, $R^3$, $R^4$, $R^5$ und X die oben angegebene Bedeutung haben, mit Anhydriden der Formel (V)

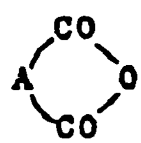

(V)

in welcher A eine gegebenenfalls substituierte Alkylenkette mit 2 oder 3 Kohlenstoffatomen oder einen Arylenrest bedeutet, zu den erfindungsgemäßen Verbindungen der Formel (Ib) umsetzt

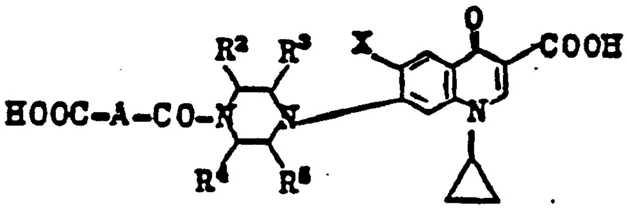

(Ib)

wobei $R^2$, $R^3$, $R^4$, $R^5$, X und A die oben angegebene Bedeutung haben.

Die als Ausgangsverbindungen verwendeten Verbindungen der Formel (II) können durch Umsetzung von Verbindungen der Formel (VI)

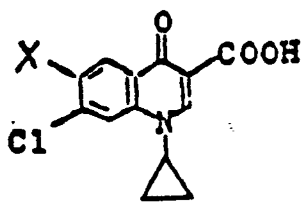

(VI)

mit Piperazin- oder Piperazinderivaten der Formel (VII)

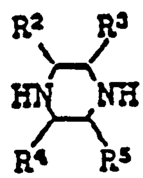

(VII)

hergestellt werden. Man arbeitet hierbei in einem Verdünnungsmittel, wie z.B. Dimethylsulfoxid, Hexamethylphosphorsäuretrisamid, Sulfolan, Wasser, einem Alkohol oder Pyridin, bei Temperaturen von 20° - 200° C, vorzugsweise bei 80° - 180° C. Bei der Durchführung des Verfahrens setzt man auf 1 Mol Carbonsäure (VI) 1 - 15 Mol der Verbindung (VII), vorzugsweise 1 - 6 Mol der Verbindung (VII) ein. Bei Verwendung äquivalenter Mengen der Carbonsäure (VI) und des Piperazinderivates (VII) führt man die Umsetzung in Gegenwart eines säurebindenden Mittels, beispielsweise Triethylamin, 1,4-Diaza-bicyclo-[2.2.2]-octan oder 1,5-Diaza-bicyclo-[5.4.0]undec-7-en durch.

Die als Zwischenprodukt verwendete 7-Chlor-1-cyclopropyl -6-fluor-1,4-dihydro-4-oxo-3-chinolincarbonsäure der Formel VIa (VI; X = F) kann gemäß folgendem Reaktionsschema hergestellt werden:

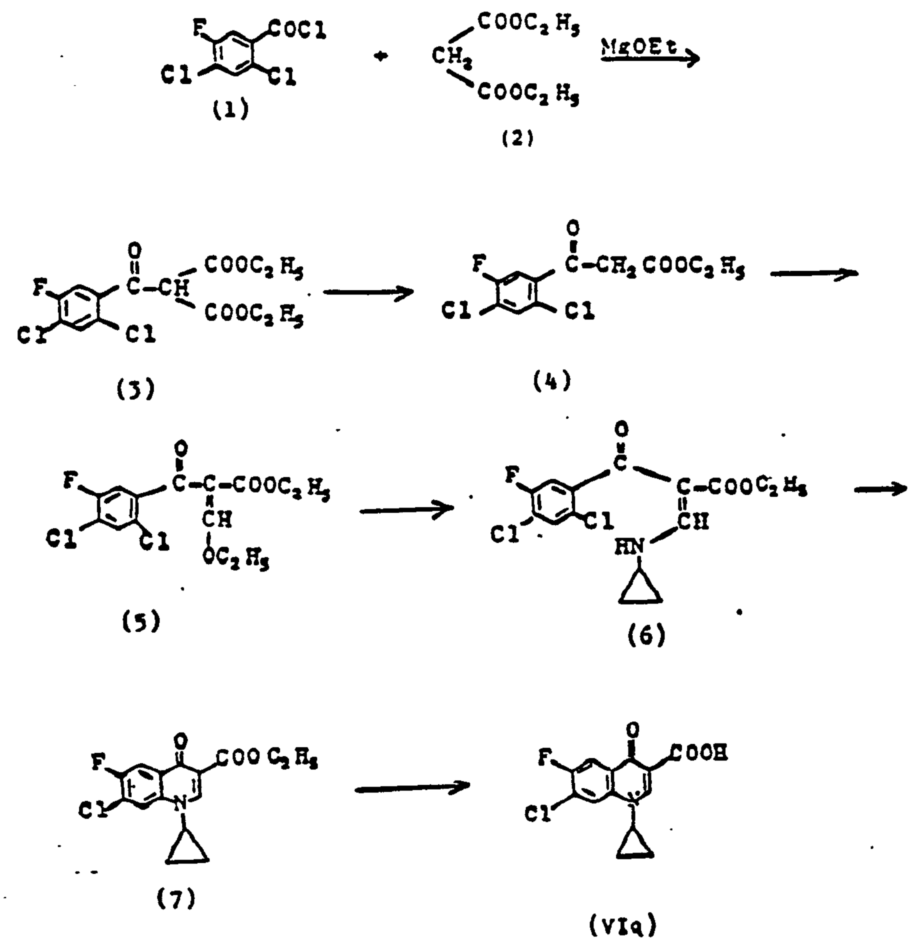


(1) (2) (3) (4) (5) (6) (7) (VIa)

Danach wird Malonsäurediethylester (2) in Gegenwart von Magnesiumalkoholat mit 2,4-Dichlor-5-fluor-benzoylchlorid (1)[Deutsche Patentanmeldung Nr. 3 142 856.8] zum Acylmalonester (3) acyliert [Organikum, 3. Aufl. 1964, S- 438].

Durch partielle Verseifung und Decarboxylierung von (3) in wäßrigem Medium mit katalytischen Mengen p-Toluolsulfonsäure erhält man in guter Ausbeute den Aroylessigsäureethylester (4), der mit o-Ameisensäuretriethylester/Acetanhydrid in den 2-(2,4-Dichlor-5-fluor-benzoyl)-3-ethoxy-acrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol, führt in leicht exothermer Reaktion zum gewünschten Zwischenprodukt (6).

Die Cyclisierungsreaktion (6) → (7) wird in einem Temperaturbereich von etwa 60° bis 280° C, bevorzugt 80° bis 180° C durchgeführt.

Als Verdünnungsmittel können Dioxan, Dimethylsulfoxid, N-Methyl-pyrrolidon, Sulfolan, Hexamethylphosphorsäuretriamid und bevorzugt N,N-Dimethylformamid verwendet werden.

Als Säurebinder kommen für diese Reaktionsstufe Kaliumtert.-butanolat, Butyl-lithium, Lithium-phenyl, Phenylmagnesiumbromid, Natriummethylat, Natriumhydrid und besonders bevorzugt Kalium- oder Natriumcarbonat in Betracht. Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zur 7-Chlor-1-cyclopropyl-6-fluor-1,4-dihydro-4-oxo-3-chinolincarbonsäure VIa.

Das als Ausgangsmaterial für diesen Syntheseweg verwendete 2,4-Dichlor-5-fluor-benzoylchlorid (1) und die entsprechende Carbonsäure sowie das für die Herstellung von

(1) benötigte 3-Fluor-4,6-dichlortoluol (10) werden gemäß nachstehendem Formelschema, ausgehend von 2,4-Dichlor-5-methyl-anilin (8), hergestellt:

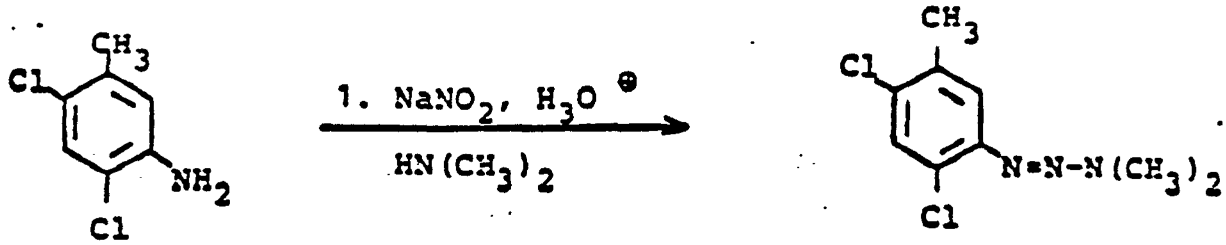


(8) (9)

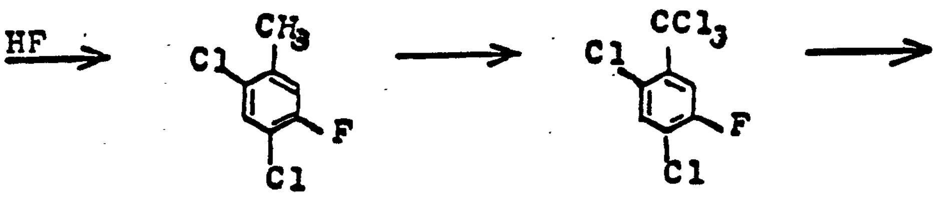


(10) (11)

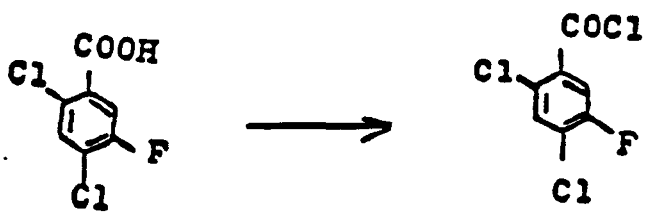

(12) (1)

Danach wird 2,4-Dichlor-5-methyl-anilin (8) mit Hilfe von $NaNO_2$ diazotiert und das dabei entstehende Diazoniumsalz mit Dimethylamin in das Triazen (9) übergeführt.

Das Triazen (9) wird in überschüssiger wasserfreier HF gelöst. Dabei spaltet das Triazen in 2,4-Dichlor-5-methyl-diazoniumfluorid und Dimethylamin. Ohne Zwischenisolierung wird diese Lösung thermisch bei 130 - 140° C unter $N_2$-Abspaltung in 3-Fluor-4,6-dichlortoluol (10) gespalten. Ausbeute: 77,7 % der Theorie.

Das 3-Fluor-4,6-dichlortoluol (10) wird in einem Temperaturbereich von 110 - 160° C unter UV-Bestrahlung zu 2,4-Dichlor-5-fluor-1-trichlormethylbenzol (11) chloriert.

Die Verseifung von (11) mit 95 %iger Schwefelsäure führt zu 2,4-Dichlor-5-fluor-benzoesäure (12), die mit Thionylchlorid in das Carbonsäurechlorid (1) (Sdp. 121°/20 mbar; $n^{20}_D$ 1,5722) übergeht.

Auf analoge Weise werden die folgenden als Zwischenprodukte verwendeten Chinoloncarbonsäuren hergestellt.

7-Chlor-1-cyclopropyl-1,4-dihydro-4-oxo-3-chinolincarbonsäure (VIb) (Schmp. 308° C) aus 2,4-Dichlorbenzoylchlorid (J. Chem. Soc. 83, 1213 (1903));

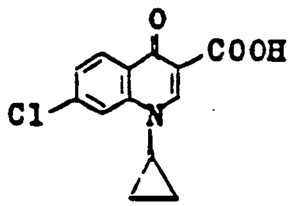

(VIb)

6,7-Dichlor-1-cyclopropyl-1,4-dihydro-4-oxo-3-chinolincarbonsäure (VIc) (Schmp. 265°C) aus 2,4,5-Trichlorbenzoylchlorid [ Liebigs Ann. Chem. 152, 238 (1896)];

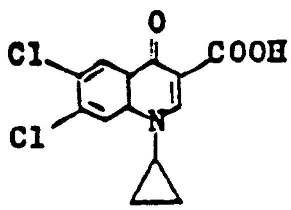

(VIc)

7-Chlor-1-cyclopropyl-1,4-dihydro-6-nitro-4-oxo-3-chinolincarbonsäure (VId) (Schmp. 265 - 275°C Zers.) aus 2,4-Dichlor-5-nitro-benzoylchlorid (Liebigs Ann. Chem. 677, 8 (1964)).

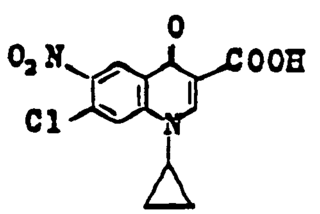

(VId)

Die als Ausgangsprodukte einsetzbaren Verbindungen der Formel (III) sind bereits bekannt.

Die als Ausgangsverbindungen einsetzbaren Isocyanate (IV) sind ebenfalls bekannt, ebenso wie die Anhydride (V).

Die Umsetzung von (II) mit (III) (Methode a) wird vorzugsweise in einem Verdünnungsmittel, wie Dimethylsulfoxid, N,N-Dimethylformamid, Tetrahydrofuran, Sulfolan, Dioxan, Pyridin, Wasser oder auch in Gemischen dieser Verdünnungsmittel, bei Temperaturen von 0°C - 140°C, vorzugsweise 10°C - 110°C, vorgenommen.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Als Säurebinder können alle üblichen anorganischen und organischen Säurebindungsmittel verwendet werden. Hierzu gehören vorzugsweise die Alkalihydroxide, Alkalicarbonate, Pyridin und tert.-Amine wie Triethylamin, 1,4-Diazabicyclo[2.2.2]octan.

Bei der Durchführung der Verfahrensvariante a) setzt man auf 1 Mol der Verbindung (II) 1 bis 4 Mol, vorzugsweise 1 bis 1,5 Mol der Verbindung (III) ein.

Die Umsetzung von (II) mit (IV) (Methode b) wird vorzugsweise in einem Verdünnungsmittel, wie z.B. Dioxan, Dimethylsulfoxid, N,N-Dimethylformamid, verdünnter Natronlauge oder auch in Gemischen dieser Verdünnungsmittel, durchgeführt.

Die Reaktionstemperaturen können in einem größeren Bereich variiert werden. Im allgemeinen arbeitet man zwischen etwa 0° C und etwa 100° C, vorzugsweise zwischen 5° C und 50° C.

Die Umsetzung kann bei Normaldruck, aber auch bei erhohtem Druck durchgeführt werden. Im allgemeinen arbeitet man bei Drucken zwischen etwa 1 und etwa 100 bar, vorzugsweise zwischen 1 und 10 bar.

Bei der Durchführung der Verfahrensvariante b) setzt man auf 1 Mol der Verbindung (II) 1 - 5 Mol, vorzugsweise 1 - 2 Mol, der Verbindung (IV) ein.

Die Umsetzung von (II) mit (V) (Methode c) wird in einem Verdünnungsmittel wie z.B. N,N-Dimethylformamid, Dioxan, Tetrahydrofuran, Pyridin, Wasser oder auch in Mischungen dieser Verdünnungsmittel, durchgeführt. Die Reaktionstemperaturen können in einem größeren Bereich variiert werden. Im allgemeinen arbeitet man zwischen etwa 0° und etwa 140° C, vorzugsweise zwischen 10° und 100° C.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Als Säurebinder können alle üblichen anorganischen und organischen Säurebindungsmittel verwendet werden. Hierzu gehören vorzugsweise die Alkalihydroxide, Alkalicarbonate, Pyridin und tert.-Amine wie Triethylamin, 1,4-Diazabicyclo[2.2.2]octan.

Bei der Durchführung der Verfahrensvariante c) setzt man auf 1 Mol der Verbindung (II) 1 bis 3 Mol, vorzugsweise 1 bis 1,3 Mol, der Verbindung (V) ein. Die erfindungsgemäß zu verwendenden Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die erfindungsgemäß zu verwendenden Wirkstoffe zeigen auch eine fungizide Wirkung, z.B. gegen Pyricularia oryzae am Reis.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol,

Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden, Insektiziden, Akariziden und Herbiziden, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

**Herstellungsbeispiele**

**I Ausgangsverbindungen**

**Beispiel A**

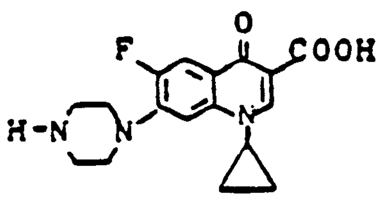

Ein Gemisch von 19,7 g 7-Chlor-1-cyclopropyl-6-fluor-1,4-dihydro-4-oxo-3-chinolincarbonsäure, 30,1 g wasserfreiem Piperazin und 100 ml Dimethylsulfoxid wird 2 Stunden auf 135 - 140°C erhitzt. Das Lösungsmittel wird im Feinvakuum abdestilliert, der Rückstand in $H_2O$ suspendiert, abgesaugt und mit Wasser gewaschen. Zur weiteren Reinigung wird das feuchte Rohprodukt mit 100 ml Wasser aufgekocht, bei Raumtemperatur abgesaugt, mit $H_2O$ gewaschen und im Vakuumtrockenschrank über $CaCl_2$ bei 100°C bis zur Gewichtskonstanz getrocknet. Man erhält 19,6 g 1-Cyclopropyl-6-fluor-1,4-dihydro-4-oxo-7-(1-piperazinyl) -3-chinolincarbonsäure vom Zersetzungspunkt 255 - 257°C.

Die als Ausgangsmaterial verwendete 7-Chlor-1-cyclopropyl -6-fluor-1,4-dihydro-4-oxo-3-chinolincarbonsäure VIa wird wie folgt hergestellt: 24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml absolutem Ethanol und 400 ml wasserfreiem Ether zu, wobei ein heftiger Rückfluß zu beobachten ist. Nach dem Abklingen der Reaktion wird noch 2 Stunden zum Sieden erhitzt, mit Trockeneis/Aceton auf -5°C bis -10°C gekühlt und bei dieser Temperatur eine Lösung von 227,5 g 2,4-Dichlor-5-fluor-benzoylchlorid (1) in 100 ml abs. Ether langsam zugetropft. Man rührt 1 Stunde bei 0°C bis -5°C, läßt über Nacht auf Raumtemperatur kommen und läßt unter Eiskühlung ein Gemisch von 400 ml Eiswasser und 25 ml konz. Schwefelsäure zulaufen. Die Phasen werden

getrennt und zweimal mit Ether nachextrahiert. Die vereinigten Etherlösungen werden mit gesättigter NaCl-Lösung gewaschen, mit $Na_2SO_4$ getrocknet und das Lösungsmittel i.Vak. abgezogen. Man erhält 349,5 g 2,4-Dichlor-5-fluor-benzoylmalonsäure -diethylester (3) als Rohprodukt.

Eine Emulsion von 34,9 g rohem 2,4-Dichlor-5-fluor-benzoyl -malonsäure-diethylester (3) in 50 ml Wasser wird mit 0,15 g p-Toluolsulfonsäure versetzt. Man erhitzt unter gutem Rühren 3 Stunden zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel i.Vak. ab. Die Fraktionierung des Rückstands i.Vak. liefert 21,8 g 2,4-Dichlor-5-fluor-benzoyl-essigsäureethylester (4) vom Sdp. 127 - 142° C/0,09 mbar.

Ein Gemisch von 21,1 g 2,4-Dichlor-5-fluor-benzoyl-essigsäureethylester (4), 16,65 g o-Ameisensäureethylester und 18,55 g Acetanhydrid wird 2 Stunden auf 150°C erhitzt. Dann werden im Wasserstrahlvakuum und zuletzt im Feinvakuum bei einer Badtemperatur von 120° C die flüchtigen Bestandteile abdestilliert. Zurück bleiben 25,2 g roher 2-(2,4-Dichlor-5-benzoyl)-3-ethoxy-acrylsäureethyl-ester (5). Es ist genügend rein für die weiteren Umsetzungen.

Eine Lösung von 24,9 g 2-(2,4-Dichlor-5-fluor-benzoyl)-3-ethoxy-acrylsäureethylester (5) in 80 ml Ethanol wird unter Eiskühlung und Rühren tropfenweise mit 4,3 g Cyclopropylamin versetzt. Wenn die exotherme Reaktion abgeklungen ist, wird noch 1 Stunde bei Raumtemperatur gerührt, das Lösungsmittel i.Vak. abgezogen und der Rückstand aus Cyclohexan/Petrolether umkristallisiert. Man erhält 22,9 g 2-(2,4-Dichlor-5-fluor-benzoyl)-3-cyclo-propylamino-acrylsäureethylester (6) vom Schmp. 89 - 90° C.

Eine Lösung von 31,9 g 2-(2,4-Dichlor-5-fluor-benzoyl)-3-cyclopropylamino-acrylsäure-ethylester (6) in 100 ml wasserfreiem Dioxan wird unter Eiskühlung und Rühren portionsweise mit 3,44 g 80-proz. Natriumhydrid versetzt. Dann wird 30 Min. bei Raumtemperatur und 2 Stunden unter Rückfluß gerührt und das Dioxan im Vakuum abgezogen. Der Rückstand (40,3 g) wird in 150 ml Wasser suspendiert, mit 6,65 g Ätzalkali versetzt und 1,5 Stunden refluxiert. Man filtriert die warme Lösung und wäscht mit $H_2O$ nach. Dann wird mit halbkonz. Salzsäure unter Eiskühlung auf pH = 1 - 2 angesäuert, der Niederschlag abgesaugt, mit Wasser gewaschen und i.Vak. bei 100°C getrocknet. Man erhält auf diese Weise 27,7 g 7-Chlor-1-cyclopropyl-6-fluor-1,4-dihydro-4-oxo-3-chinolincarbonsäure VIa vom Schmp. 234 - 237° C.

**Beispiel B**

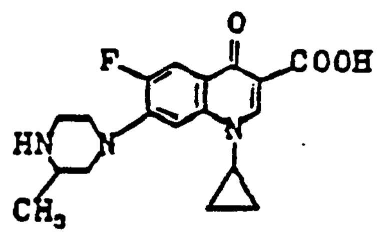

x 1/2 $H_2O$

Ein Gemisch aus 2,8 g (0,01 Mol) 7-Chlor-1-cyclopropyl-6-fluor-1,4-dihydro-4-oxo-3-chinolincarbonsäure und 5,1 g (0,051 Mol) 2-Methylpiperazin in 6 ml Dimethylsulfoxid wird 2 Stunden auf 140° C erhitzt. Anschließend wird das Lösungsmittel im Hochvakuum abdestilliert, der Rückstand mit 6 ml heißem Wasser versetzt und 1 Stunde auf 95°C gehalten. Man kühlt mit Eis, saugt den ausgefallenen Niederschlag ab, wäscht mit etwas Wasser und löst ihn in einer Mischung von 0,8 ml Essigsäure und 10 ml Wasser bei 90 - 100°C auf. Das Filtrat wird mit Kalilauge (0,75 g KOH in 0,7 ml Wasser) auf pH 8 gebracht und der ausgefallene Niederschlag aus Methanol umkristallisiert. Man erhält 1,8 g (52 % der Theorie) 1-Cyclopropyl-6-fluor-1,4-dihydro-4-oxo-7-(3-methyl-1-piperazinyl)-3-chinolincarbonsäure-semihydrat mit einem Zersetzungspunkt von 230 - 232° C.

**Beispiel C**

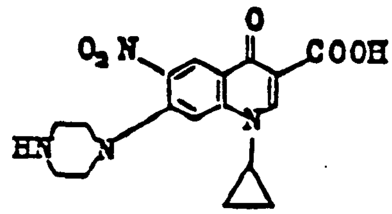

Eine Mischung von 9,3 g (0,03 Mol) 7-Chlor-1-cyclopropyl-1,4-dihydro-6-nitro-4-oxo-3-chinolincarbonsäure und 12,9 g (0,15 Mol) Piperazin wird in 60 ml Dimethylsulfoxid 15 Minuten auf 120°C erwärmt. Aus der heißen Lösung fällt nach kurzer Zeit ein Niederschlag aus. Man engt im Hochvakuum ein, verrührt mit 30 ml Wasser und erhitzt noch 30 Minuten auf 95°C. Mit 2n-Salzsäure wird die Mischung auf pH 8 eingestellt, der Niederschlag abgesaugt und mit Wasser und Methanol gewaschen. Man isoliert 5,8 g (54 % d.Th.) 1-Cyclopropyl-1,4-dihydro-6-nitro-4-oxo-7-(1-piperazinyl) -3-chinolincarbonsäure mit einem Zersetzungspunkt von 296 - 298°C

**Beispiel D**

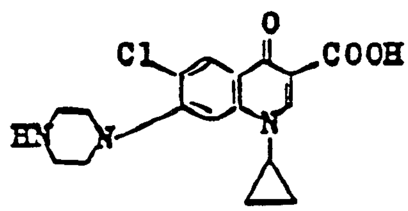

Man setzt analog Beispiel C 6,7-Dichlor-1-cyclopropyl-1,4-dihydro-4-oxo-3-chinolincarbonsäure zu 1-Cyclopropyl -6-chlor-1,4-dihydro-4-oxo-7-(1-piperazinyl)-3-chi-nolincarbonsäure mit einem Zersetzungspunkt von 295-298°C um.

**Beispiel E**

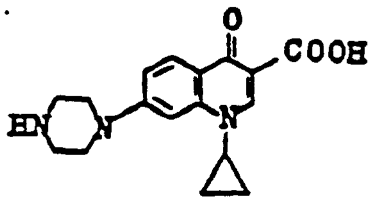

Man setzt analog Beispiel C 7-Chlor-1-cyclopropyl-1,4-dihydro-4-oxo-3-chinolincarbonsäure mit Piperazin zu 1-Cyclopropyl-1,4-dihydro-4-oxo-7-(1-piperazinyl)-3-chinolincarbonsäure mit einem Zersetzungspunkt von 298-300° C um.

**II Endprodukte**

**Beispiel 1**

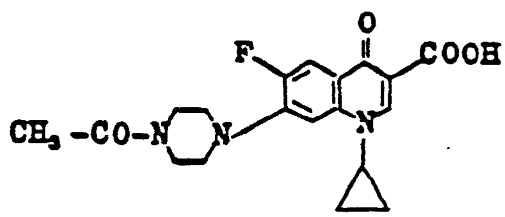

3,3 g (0,01 Mol) 1-Cyclopropyl-6-flour-1,4-dihydro-4-oxo-7-(1-piperazinyl)-3-chinolincarbonsäure wird in einer Mischung aus 20 ml Dioxan und einer Lösung von 0,4 g Natriumhydroxid in 5 ml Wasser gelöst. Hierzu tropft man unter Eiskühlung gleichzeitig eine Lösung von 0,9 g (0,011 Mol) Acetylchlorid in 5 ml Dioxan und eine Lösung von 0,4 g (0,01 Mol) Natriumhydroxid in 5 ml Wasser zu, wobei der pH-Wert >8 gehalten wird. Man läßt 2 Stunden bei Raumteperatur nachrühren, versetzt die Suspension mit 30 ml Wasser, säuert mit 2n Salzsäure an, saugt den Niederschlag ab und kristallisiert aus Glykolminimethylether um. Es werden 2 g (54 % der Theorie) 7-(4-Acetyl-1-piperazinyl)-1-cyclopropyl-6-flour-1,4-dihydro-4-oxo-3-chinolincarbonsäure mit einem Zersetzungspunkt von 267 - 270° C isoliert.

Entsprechend Beispiel 1 weden die folgenden Verbindungen erhalten:

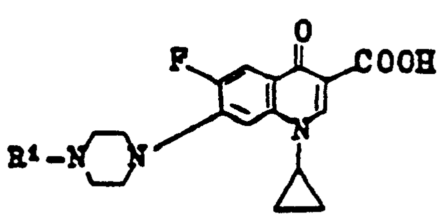

| Beispiel | $R^1$ | Schmelzpunkt [°C] |
|---|---|---|
| 2 | $CH_3-CH_2-CO$ | 267 (Zers.) |
| 3 | $CH_3-CH_2-CH_2-CO$ | 282 (Zers.) |
| 4 | 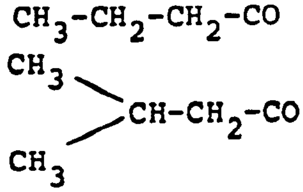 | 286 (Zers.) |
| 5 | $CF_3-S-CH_2-CO$ | 252 (Zers.) |
| 6 | 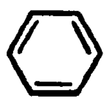-CO- | 324 (Zers.) |
| 7 | $CH_3O-CO-CH_2-CH_2-CO$ | 205 |
| 8 | CN | 276 (Zers.) |
| 9 | $CH_3O-CO$ | 270 (Zers.) |
| 10 | $C_2H_5OCO$ | 315 (Zers.) |
| 11 | $n-C_4H_9OCO$ | 245 |
| 12 | 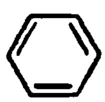$-CH_2-O-CO-$ | 230 |
| 12a | $C_2H_5-S-CO-$ | 322 (Zers.) |
| 13 | $CH_3-SO_2-$ | 305 (Zers.) |
| 14 | $n-C_3H_7-SO_2-$ | 268 (Zers.) |
| 15 | $CFCl_2-SO_2-$ | 278 (Zers.) |
| 16 | $CCl_3-S-$ | 172 (Zers.) |
| 17 | $CFCl_2-S-$ | 188 (Zers.) |
| 18 | $CH_3O-CO-S-$ | 204 (Zers.) |
| 19 | $CH_3-CH_2-CH_2-O-CH_2-CO-$ | 220 |

**Beispiel 20**

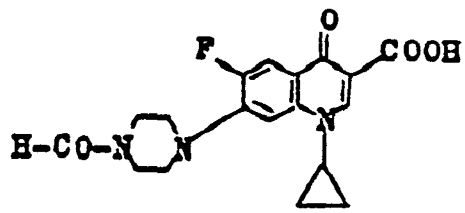

Man arbeitet wie in Beispiel 1 unter Werwendung von Ameisensäure-essigsäure-anhydrid und erhält 1-Cyclopropyl -6-fluor-7-(4-formyl-1-piperazinyl)-1,4-dihydro-4-oxo-3-chinolincarbonsäure mit einem Zersetzungspunkt von 278 - 281°C.

**Beispiel 21**

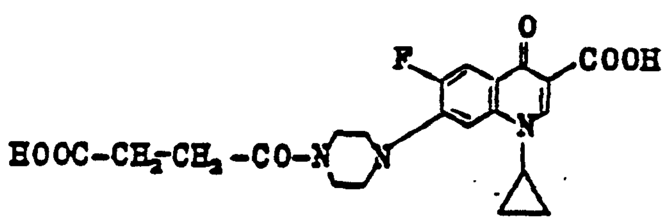

3,3 g (0 01 Mol) 1-Cyclopropyl-6-fluor-1,4-dihydro-4-oxo-7-(1-piperazinyl)-3-chinolincarbonsäure werden in einer Lösung von 0,4 g Natriumhydroxid in 20 ml Wasser gelöst und bei Raumtemperatur gleichzeitig mit einer Lösung von 1 g Bernsteinsäureanhydrid in 10 ml Dioxan und 0,4 g Natriumhydroxid in 10 ml Wasser versetzt. Man läßt 2 Stunden bei Raumtemperatur nachrühren, säuert mit 2n-Salzsäure an, saugt den ausgefallenen Niederschlag ab und wäscht ihn mit Wasser und Methanol. Es werden 3,4 g (79 % der Theorie) 7-[4-(3-Carboxypro-pionyl)-1-piperazinyl]-1-cyclopropyl-6-fluor-1,4-dihydro -4-oxo-3-chinolincarbonsäure mit einem Zersetzungspunkt von 284 - 286°C erhalten.

Entsprechend Beispiel 21 werden die folgenden Verbindungen erhalten:

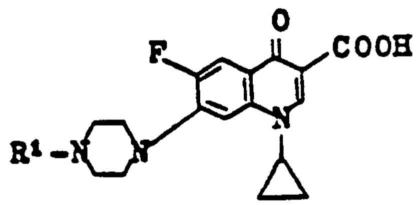

| Beispiel | $R^1$ | Schmelzpunkt [°C] |
|---|---|---|
| 22 | $HOOC-CH_2-CH_2-CH_2-CO-$ | 273 |
| 23 | 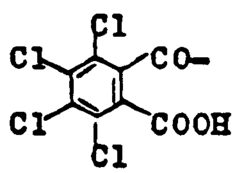 | 253 (Zers.) |

**Beispiel 24**

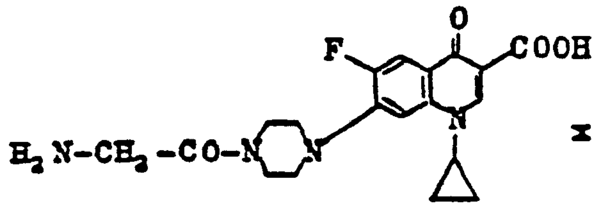

x 1/2 $H_2O$

x 1/2 $H_2O$

3,3 g (0 01 Mol) 1-Cyclopropyl-6-fluor-1,4-dihydro-4-oxo-7-(1-piperazinyl)-3-chinolincarbonsäure werden in 50 ml Pyridin mit 3 g N-(tert.-Butoxycarbonyl)-glycin-4-nitro-phenylester versetzt und 4 Stunden bei Raumtemperatur gerührt. Die Lösung wird eingeengt, mit 30 ml Wasser versetzt und mit 2n-Salzsäure auf pH 5 eingestellt. Der ausgefallene Niederschlag wird abgesaugt, mit Wasser und Methanol gewaschen und getrocknet. Man erhält 2,7 g (55 % der Theorie) 7-[4-(tert.-Butoxycarbonylamino-acetyl)-1-piperazinyl]-1-cyclopropyl-6-fluor-1,4-dihydro-4-oxo-3-chinolincarbonsäure mit einem Zersetzungspunkt von 236 - 238° C. Eine Suspension von 2,2 g (0 0045 Mol) dieses Zwischenproduktes in 100 ml Methanol wird mit 10 ml konzentrierter Salzsäure versetzt und 3 Stunden bei Raumtemperatur stehengelassen. Danach werden Methanol und überschüssiger Chlorwasserstoff im Vakuum abgezogen, die wäßrige Lösung mit verdünnter Natronlauge auf pH 8 eingestellt, der Niederschlag abgesaugt und mit Methanol gewaschen. Es werden 1,4 g (78 % der Theorie) 7-[4(Aminoacetyl)-1-piperazinyl]-1-cyclopropyl-6-fluor-1,4-dihydro-4-oxo-3-chinolincarbonsäure-semihydrat mit einem Zersetzungspunkt von 245 - 248° C erhalten.

Analog Beispiel 24 werden folgende Verbindungen hergestellt:

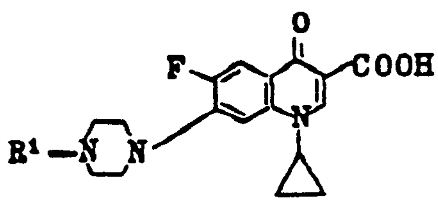

| Beispiel | $R^1$ | Zersetzungspunkt [°C] |
|---|---|---|
| 25 | 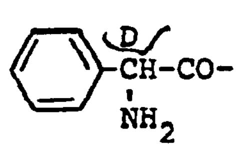 x HCl x $H_2O$ | 297 |
| 26 | 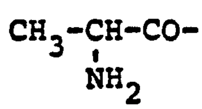 | 240 |

| Beispiel | $R^1$ | Zersetzungspunkt [°C] |
| --- | --- | --- |
| 27 | 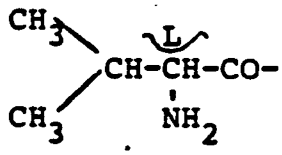 x HCl | 280 |
| 28 | $H_2N-CH_2-CH_2-CO-$ x HCl | 274 |

**Beispiel 29**

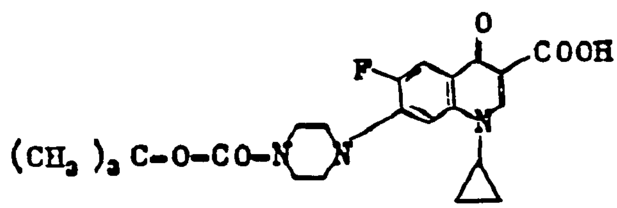

Eine Lösung von 8,25 g (0,025 Mol) 1-Cyclopropyl-6-fluor-1,4-dihydro-4-oxo-7-(1-piperazinyl)-3-chinolincarbonsäure in 75 ml Dioxan/Wasser (2:1) und 25 ml 1n-Natronlauge wird unter Eiskühlung mit 6 g Pyrokohlensäure-di-tert.-butylester versetzt und die Mischung dann 30 Minuten bei Raumtemperatur gerührt. Man engt bis auf ein Drittel des Volumens ein, überschichtet mit 50 ml Essigsäureethylester und säuert mit verdünnter Kaliumhydrogensulfatlösung bis pH 3 an. Der ausgefallene Niederschlag wird abgesaugt, mit Wasser und Methanol gewaschen und im Hochvakuum getrocknet. Man erhält 10 g (92 % der Theorie) 7-(4-tert.-Butoxycarbonyl-1-piperazinyl)-1-cyclopropyl-6-fluor-1,4-dihydro-4-oxo-3-chinolincarbonsäure mit einem Zersetzungspunkt von 249 - 252° C.

**Beispiel 30**

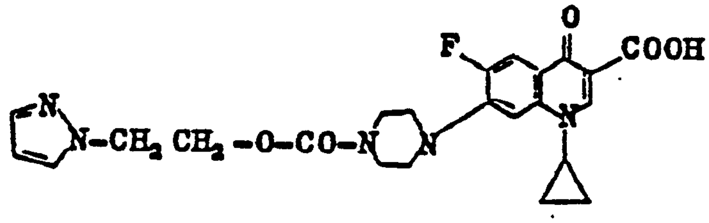

Eine Mischung von 3,3 g (0,01 Mol) 1-Cyclopropyl-6-fluor-1,4-dihydro-4-oxo-7-(1-piperazinyl)-3-chinolincarbonsäure und 2,8 g (0,01 Mol) 4-Nitrophenyl-[2-(1-pyrazolyl)-ethyl]-carbonat in 40 ml Pyridin wird 1 Tag bei Raumtemperatur gerührt. Danach wird mit 30 ml Wasser verdünnt, mit 2n-Salzsäure angesäuert, der Niederschlag abgesaugt und aus Glykolmonomethylether umkristallisiert. Man erhält 2,8 g (60 % der Theorie) 1-Cyclopropyl-6-fluor-1,4-dihydro-4-oxo-7-{ 4-[2-(1-pyrazolyl) -ethyloxycarbonyl]-1-piperazinyl} -3-chinolincarbonsäure mit einem Schmelzpunkt von 205 - 208° C.

Das als Ausgangsprodukt eingesetzte 4-Nitrophenyl-[2-(1-pyrazolyl)-ethyl]-carbonat wird auf folgendem Wege erhalten: 4,6 g 1-(2-Hydroxyethyl)-pyrazol werden in 80 ml Acetonitril mit 4 g Chlorkohlensäure-4-nitrophenylester bei Raumtemperatur 12 Stunden gerührt, die Lösung eingeengt, das erhaltene Öl in Methylenchlorid aufgenommen und mit Wasser gewaschen. Man trocknet mit Natriumsulfat, engt ein und erhält das rohe Carbonat als zähes Öl.

Analog Beispiel 30 werden die folgenden Verbindungen erhalten:

R-O-CO-

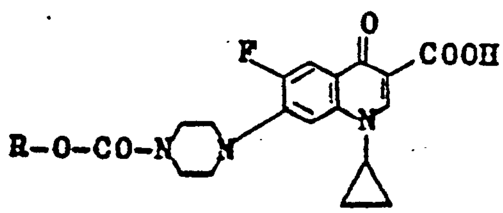

| Beispiel | R | Schmelzpunkt [°C] |
|---|---|---|
| 31 | 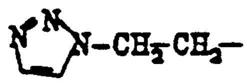 | 226 |
| 32 | 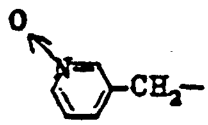 | 236 (Zers.) |
| 33 | 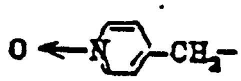 | 203 (Zers.) |

**Beispiel 34**

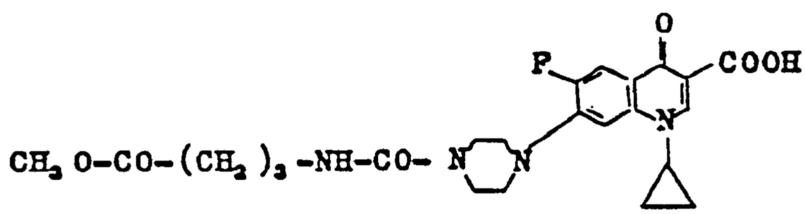

6,6 g (0,02 Mol) 1-Cyclopropyl-6-fluor-1,4-dihydro-4-oxo-7-(1-piperazinyl)-3-chinolincarbonsäure werden in einer Lösung von 0,4 g Natriumhydroxid in 40 ml Wasser gelöst und unter Eiskühlung mit einer Lösung von 3,2 g (0,022 Mol) 4-Isocyanato-buttersäuremethylester in 12 ml Dioxan versetzt. Danach wird 2 Stunden bei Raumtemperatur nachgerührt, mit 2n Salzsäure auf pH 5 eingestellt, der Niederschlag abgesaugt und aus Methanol umkristallisiert. Man erhält 6 g (63 % der Thorie) 1-Cyclopropyl-6-fluor-1,4-dihydro-4-oxo-7-{4-[(3-methoxycarbonylpropyl) -carbamoyl]-1-piperazinyl} -3-chinolincarbonsäure mit einem Schmelzpunkt von 198 - 200°C.

Entsprechend Beispiel 34 werden die folgenden Verbindungen erhalten:

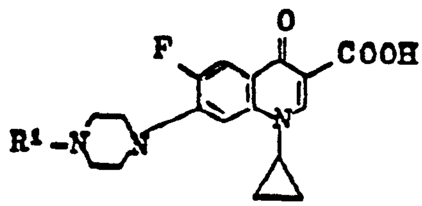

| Beispiel | $R^1$ | Schmelzpunkt (°C) |
|---|---|---|
| 35 | $CH_3O-CO(CH_2)_5-NH-CO-$ | 178 |
| 36 | $C_3H_7O-CO-(CH_2)_2-NH-CO-$ | 180 (Zers.) |
| 37 | $CH_3-NH-CO-$ | 280 (Zers.) |

**Beispiel 38**

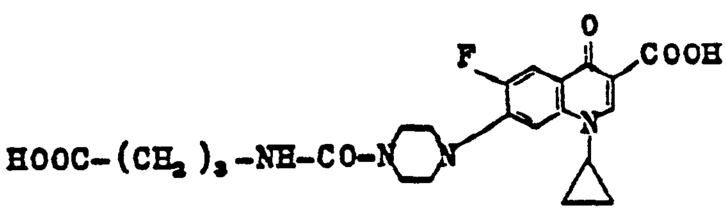

2,37 g der Verbindung des Beispiels 34 werden in einer Mischung aus 10 ml Eisessig, 6,5 ml Wasser und 1 ml konzentrierter Schwefelsäure 1,5 Stunden auf 150 - 160°C erhitzt. Nach dem Abkühlen auf Raumtemperatur wird auf 50 ml Eiswasser ausgegossen, der Niederschlag abgesaugt und aus Glykolmonomethylether umkristallisiert. Man isoliert 1,2 g (52 % der Theorie) 7-{4-[(3-Carboxy-propyl)-carbamoyl]-1-piperazinyl}-1-cyclopropyl-6-fluor-1,4-dihydro -4-oxo-3-chinolincarbonsäure mit einem Zersetzungspunkt von 245 - 248°C.

Auf entsprechende Weise wie in Beispiel 38 werden folgende Verbindungen durch Hydrolyse der Produkte aus den Beispielen 35 und 36 erhalten:

$HOOC-(CH_2)_n-NH-CO-$

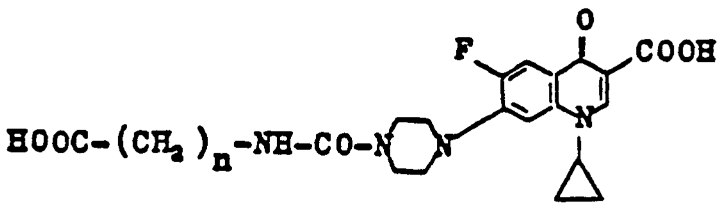

| Beispiel | n | Schmelzpunkt (°C) |
|---|---|---|
| 39 | 5 | 224 (Zers.) |
| 40 | 2 | 209 (Zers.) |

**Beispiel 41**

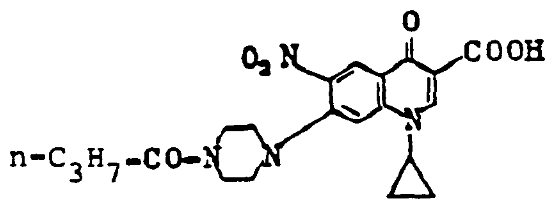

Entsprechend Beispiel 1 werden 1-Cyclopropyl-1,4-dihydro -6-nitro-4-oxo-7-(1-piperazinyl)-3-chinolincarbonsäure und Buttersäurechlorid zu 7-(4-Butyryl-1-piperazinyl) -1-cyclopropyl-1,4-dihydro-6-nitro-4-oxo-3-chinolincarbonsäure mit einem Zersetzungspunkt von 223-226° C umgesetzt.

**Beispiel 42**

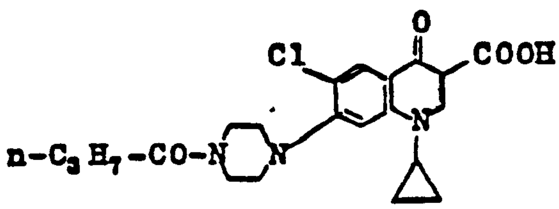

Entsprechend Beispiel 1 werden 6-Chlor-1-cyclopropyl-1,4-dihydro-4-oxo-7-(1-piperazinyl)-3-chinolincarbonsäure und Buttersäurechlorid zu 7-(4-Butyryl-1-piperazinyl) -6-chlor-1-cyclopropyl-1,4-dihydro-4-oxo-3-chinolincarbonsäure mit einem Zersetzungspunkt von 280 - 283° C umgesetzt.

**Beispiel 43**

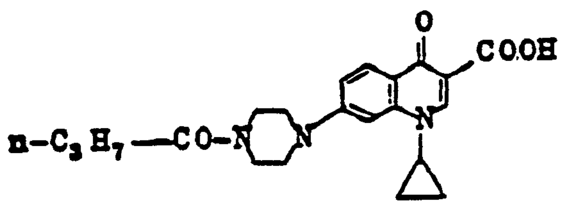

Entsprechend Beispiel 1 werden 1-Cyclopropyl-1,4-dihydro-4-oxo-7-(1-piperazinyl)-3-chinolincarbonsäure und Buttersäurechlorid zu 7-(4-Butyryl-1-piperazinyl)-1-cyclopropyl-1,4-dihydro-4-oxo-3-chinolincarbonsäure mit einem Zersetzungspunkt von 252-255° C umsetzt.

**Beispiel 44**

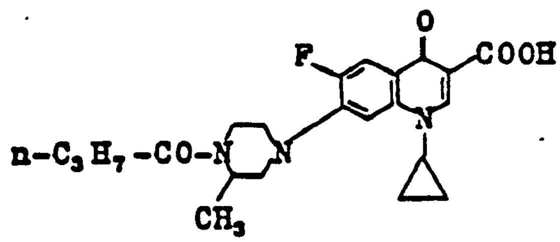

Entsprechend Beispiel 1 werden 1-Cyclopropyl-6-fluor-1,4-dihydro-4-oxo-7-(3-methyl-1-piperazinyl)-3-chinolincarbonsäure und Buttersäurechlorid zu 7-(4-Butyryl -3-metyl-1-piperazinyl)-1-cyclopropyl-6-flour-1,4-dihydro-4-oxo-3-chinolincarbonsäure mit einem Zersetzungspunkt von 226-228° C umgesetzt.

**Verwendungsbeispiele**

In den nachfolgenden Beispielen wird die folgende Verbindung als Vergleichssubstanz eingesetzt:

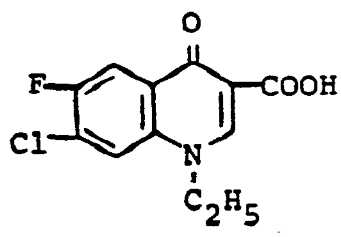

**Beispiel I**

Xanthomonas oryzae-Test / Bakteriose / Reis / systemisch
Lösungsmittel: 48,5 Gewichtsteile Dimethylformamid
Emulgator: 1,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften werden 100 ml der Wirkstoffzubereitung auf Einheitserde gegossen, in der junge Pflanzen angezogen wurden. 3 Tage nach der Behandlung werden die Pflanzen mit einer wäßrigen Suspension von Xanthomonas oryzae durch Stichverletzung inokuliert. Danach verbleiben die Pflanzen 14 Tage bis zur Auswertung in einem Gewächshaus bei 24 bis 26°C und 70 bis 80 % rel. Luftfeuchtigkeit.

Eine deutliche Überlegenheit in der Wirksamkeit gegenüber dem Stand der Technik zeigen bei diesem Test z.B. die Verbindungen gemäß folgender Herstellungsbeispiele:

**Tabelle I**

Xanthomonas oryzae-Test / Bakteriose / Reis / systemisch
Wirkstoffe Aufwand- Krankheitsbe
menge in fall in % der

## T a b e l l e  I

Xanthomonas oryzae-Test / Bakteriose / Reis / systemisch

| Wirkstoffe | Aufwandmenge in mg Wirkstoff pro 100 cm² | Krankheitsbefall in % der unbehandelten Kontrolle systemisch |
|---|---|---|
| 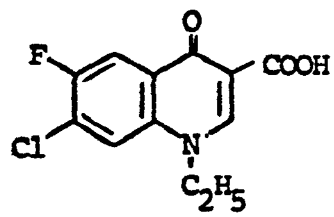 (bekannt) | 10 | 60 |
| 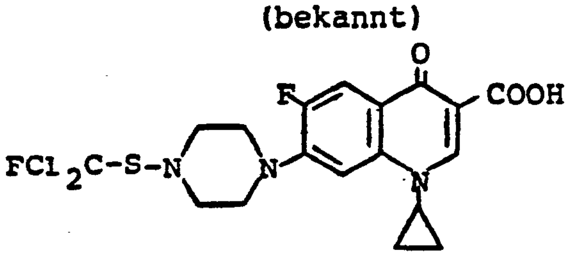 | 10 | 40 |
| 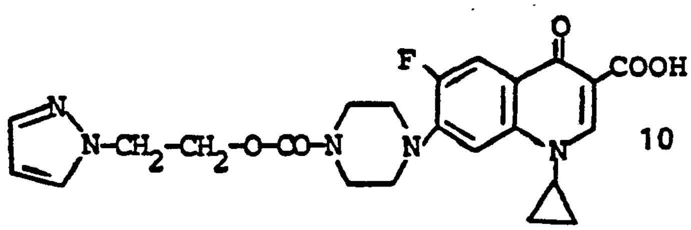 | 10 | 50 |
| 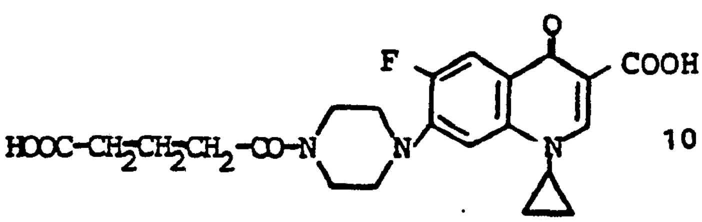 | 10 | 40 |

Tabelle I (Fortsetzung)

Xanthomonas oryzae-Test / Bakteriose / Reis / systemisch

| Wirkstoffe | Aufwandmenge in mg Wirkstoff pro 100 $cm^2$ | Krankheitsbefall in % der unbehandelten Kontrolle systemisch |
|---|---|---|
| 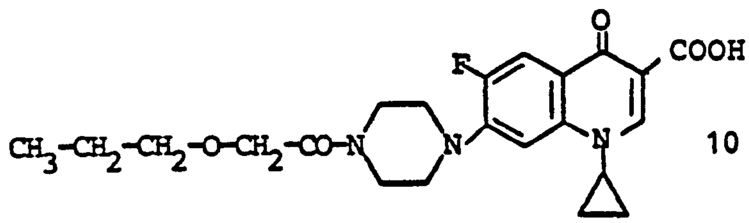 | 10 | 50 |

**Beispiel II**

**Agarplattentest**

Verwendeter Nährboden
15 Gewichtsteile Agar-Agar
10 Gewichtsteile Saccharose
8 Gewichtsteile Caseinhydrolysat
4 Gewichtsteile Hefeextrakt
2 Gewichtsteile Dikaliumhydrogenphosphat
0,3 Gewichtsteile Magnesiumphosphat
werden in 1000 ml destilliertem Wasser gelöst und 15 Minuten bei 121°C autoklaviert.
Lösungsmittel: 10 Gewichtsteile Dimethylformamid
Mengenverhältnis von Lösungsmittel zu Nährboden: 0,2:99,8

Zur Herstellung einer zweckmäßigen Wirkstoffzubereitung vermischt man 1 Gewichtsteil Wirkstoff mit der angegebenen Menge Lösungsmittel.

Das Konzentrat wird im genannten Mengenverhältnis mit dem flüssigen Nährboden gründlich-vermischt und in Petrischalen gegossen.

Ist der Nährboden erkaltet und fest, werden die Platten mit folgenden Mikroorganismen beimpft und bei ca. 28°C inkubiert:

Die Auswertung erfolgt nach 2 Tagen, wobei die Wachstumshemmung als Maß für die Wirkung der Präparate herangezogen wird.

Eine deutliche Überlegenheit gegenüber dem Stand der Technik zeigen in diesem Test die Verbindungen gemäß folgender Herstellungsbeispiele:

**T a b e l l e II**

Agarplatten-Test

| Wirkstoffe | Wirkstoffkonzentration ppm | Wachstum in Wertzahlen (1=kein Wachstum, 9=Kontrolle) Mikroorganismen: Agrobacterium tumefaciens | Corynebacterium michiganense |
|---|---|---|---|
| 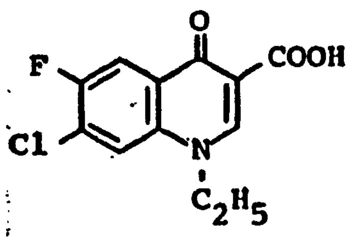 (bekannt) | 10 | 4 | 4 |
| 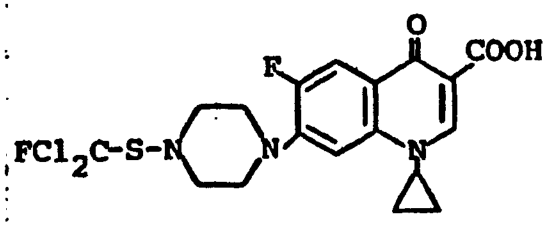 | 10 | 2 | 2 |
| 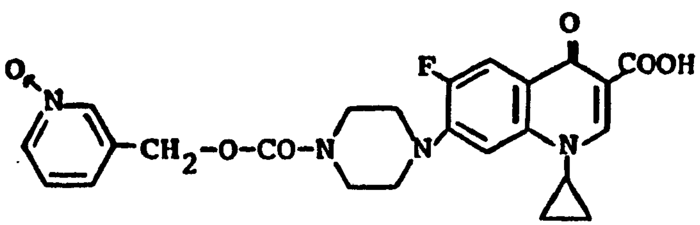 | 10 | 2 | 2 |

**Tabelle II** (Fortsetzung)

Agarplatten-Test

| Wirkstoffe | Wirkstoff-konzentration ppm | Wachstum in Wertzahlen (1=kein Wachstum, 9=Kontrolle) Mikroorganismen: Agrobacterium tumefaciens | Corynebacterium michiganense |
|---|---|---|---|
| 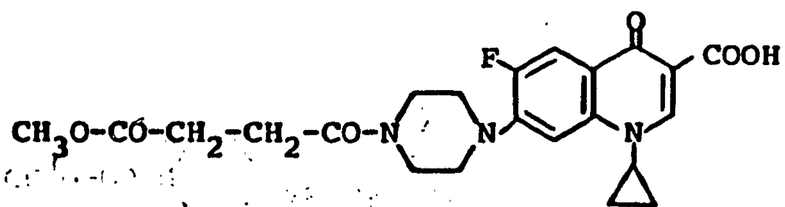 | 10 | 3 | 2 |
| 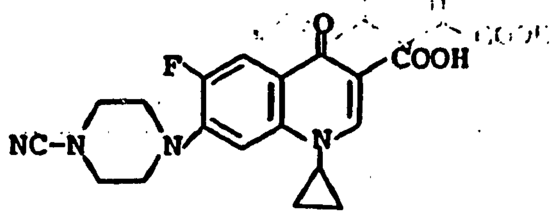 | 10 | 3 | 2 |
| 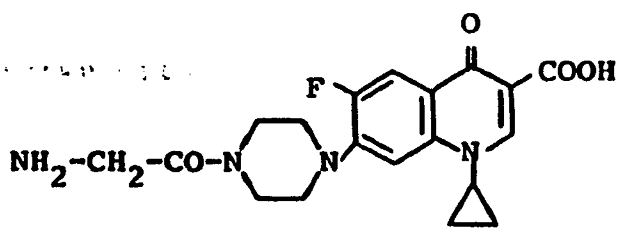 x HCl | 10 | 3 | 2 |

**T a b e l l e II** (Fortsetzung)

Agarplatten-Test

| Wirkstoffe | Wirkstoff-konzentration ppm | Wachstum in Wertzahlen (1=kein Wachstum, 9=Kontrolle) Mikroorganismen: Agrobacterium tumefaciens | Corynebacterium michiganense |
|---|---|---|---|
| 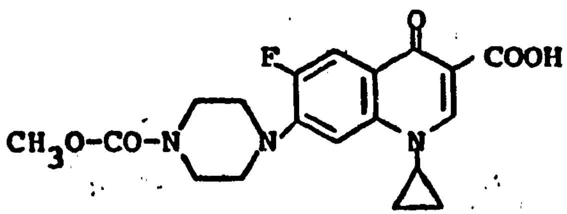 | 10 | 3 | 2 |
| 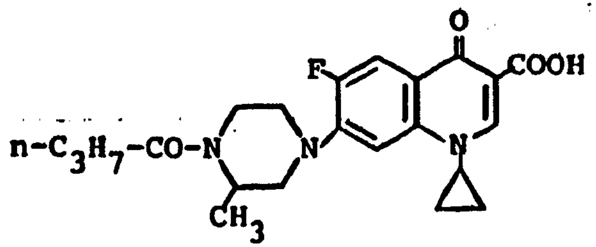 | 10 | 3 | 3 |
| 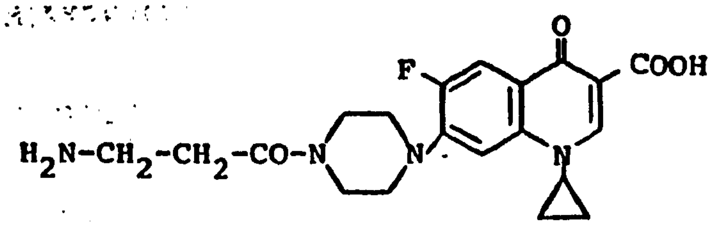 x HCl | 10 | 3 | 2 |

**Patentansprüche**

1. Verwengung von 1-Cyclopropyl-1,4-dihydro-4-oxo-3-chinolincarbonsäure-Derivaten der Formel (I)

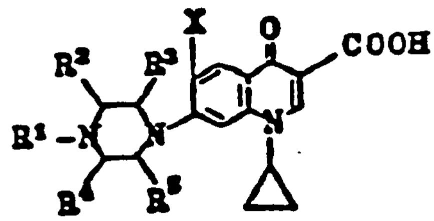

(I)

in der

$R^1$ für einen Rest $CO-R^6$, CN, $SO_2-R^7$ oder $S-R^8$ steht, wobei

$R^6$ für Wasserstoff, gegebenenfalls substituiertes geradkettiges oder verzweigtes Alkyl mit 1 bis 6 Kohlenstoffatomen, gegebenenfalls substituiertes Aryl mit 6 oder 10 Kohlenstoffatomen, gegebenenfalls substituiertes Aralkyl mit 6 Kohlenstoffatomen im Arylteil und 1 bis 3 Kohlenstoffatomen in Alkylteil, gegebenenfalls substituiertes Alkoxy oder Alkylthio mit 1 bis 6 Kohlenstoffatomen, gegebenenfalls substituiertes Phenoxy gegebenenfalls substituiertes Benzyloxy, Amino, gegebenenfalls substituiertes Alkylamino oder Dialkylamino mit 1 bis 6 Kohlenstoffatomen je Alkylrest oder gegebenenfalls substituiertes Phenylamino steht,

$R^7$ für gegebenenfalls substituiertes geradkettiges oder verzweigtes Alkyl mit 1 bis 5 Kohlenstoffatomen, Phenyl oder Methylphenyl steht,

$R^8$ für Methoxycarbonyl, Trichlormethyl, Trifluormethyl oder Dichlorfluormethyl steht,

$R^2$, $R^3$, $R^4$ und $R^5$ gleich oder verschieden sein können und für Wasserstoff, Methyl, Ethyl, n- oder i-Propyl stehen, und

X für Wasserstoff, Halogen oder Nitro steht, und deren Säureadditions-, Alkali- und Erdalkalisalze, Zink-, Mangan- und Kupfersalze und Hydrate zur Bekämpfung von Bakterien im Pflanzenschutz.

2. Verwendung der Verbindungen der Formel (I) gemäß Anspruch 1, in der

$R^1$ für $CO-R^6$, CN, $SO_2-R^7$ oder $S-R^8$ steht, wobei

$R^6$ für Wasserstoff, für gegebenenfalls ein- bis dreifach durch Amino, Chlor, Alkoxycarbonyl mit 1 bis 4 Kohlenstoffatomen im Alkylteil, Carboxy, Alkoxy mit 1 bis 4 Kohlenstoffatomen, Hydroxy oder Trifluormethylthio substituiertes geradkettiges oder verzweigtes Alkyl mit 1 bis 5 Kohlenstoffatomen, gegebenenfalls ein- bis fünffach durch Fluor, Chlor, Hydroxy, Methoxy, Amino, Nitro oder Carboxy substituiertes Phenyl, für gegebenenfalls durch Amino im Methylenrest substituiertes Benzyl, gegebenebenfalls durch Fluor, Chlor, Pyrazol-1-yl, 1, 2, 3-Triazol-1-yl, N-Oxido-2-, -3- oder -4-pyridyl substituiertes Alkoxy oder Alkylthio mit 1 bis 5 Kohlenstoffatomen, für Benzyloxy, Amino, gegebenenfalls durch Alkoxycarbonyl mit 1 bis 3 Kohlenstoffatomen im Alkylteil, Benzyloxycarbonyl, Carboxy oder Halogen substituiertes Alkylamino mit 1 bis 5 Kohlenstoffatomen oder für Phenylamino steht,

$R^7$ für gegebenenfalle ein- bis dreifach durch Fluor, Chlor oder Amino substituiertes geradkettiges oder verzweigtes Alkyl mit 1 bis 4 Kohlenstoffatomen, für Phenyl oder Methylphenyl steht,

$R^8$ für Methoxycarbonyl, Trichlormethyl oder Dichlorfluormethyl steht, und

$R^2$, $R^3$, $R^4$ und $R^5$ Wasserstoff, Methyl oder Ethyl und

X Wasserstoff, Fluor, Chlor oder Nitro

bedeuten, zur Bekämpfung von Bakterien im Pflanzenschutz.

3. Verwendung der Verbindungen der Formel (I) gemäß Anspruch 1, in der

$R^1$ für $CO-R^6$, CN, $SO_2-R^7$ oder $S-R^8$ steht,

$R^6$ für Wasserstoff, gegebenenfalls ein- oder zweifach durch Amino, Alkoxycarbonyl mit 1 bis 3 Kohlenstoffatomen im Alkylteil, Carboxy, Alkoxy mit 1 bis 3 Kohlenstoffatomen oder Trifluormethylthio substituiertes geradkettiges oder verzweigtes Alkyl mit 1 bis 4 Kohlenstoffatomen, gegebenenfalls ein- bis fünffach durch Chlor, Hydroxy, Amino oder Carboxy substituiertes Phenyl, gegebenenfalls durch Amino im Methylenrest substituiertes Benzyl, gegebenenfalls durch Pyrazol-1-yl, 1,2,3-Triazol-1-yl, N-Oxido-2-, -3- oder -4-pyridyl substituiertes Alkoxy mit 1 bis 4 Kohlenstoffatomen oder Alkylthio mit 1 oder 2 Kohlenstoffatomen, für Benzyloxy, Amino, gegebenenfalls durch Alkoxycarbonyl mit 1 bis 3 Kohlenstoffatomen im Alkylteil, Chlor oder durch Carboxy substituiertes Alkylamino mit 1 bis 5 Kohlenstoffatomen steht,

$R^7$ für geradkettiges oder verzweigtes Alkyl mit 1 bis 3 Kohlenstoffatomen, Dichlorfluormethyl, Chlorbutyl, Phenyl oder Methylphenyl steht,

$R^8$ für Methoxycarbonyl, Trichlormethyl oder Dichlorfluormethyl steht,

$R^2$ für Wasserstoff, Methyl oder Ethyl steht,
$R^3$ für Wasserstoff steht,
$R^4$ für Wasserstoff oder Methyl steht,
$R^5$ für Wasserstoff
und
X für Wasserstoff, Fluor, Chlor oder Nitro
stehen, zur Bekämpfung von Bakterien im Pflanzenschutz.

4. Zink-, Mangan- oder Kupfersalze von 1-Cyclopropyl-1,4-dihydro-4-oxo-3-chinolincarbonsäure-Derivaten der Formel (I)

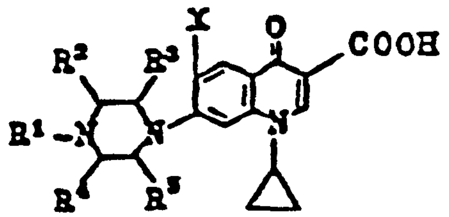

(I)

in der
$R^1$ für einen Rest CO-$R^6$ CN, $SO_2$-$R^7$ oder S-$R^8$ steht, wobei
$R^6$ für Wasserstoff, gegebenenfalls substituiertes geradkettiges oder verzweigtes Alkyl mit 1 bis 6 Kohlenstoffatomen, gegebenenfalls substituiertes Aryl mit 6 oder 10 Kohlenstoffatomen, gegebenenfalls substituiertes Aralkyl mit 6 Kohlenstoffatomen im Arylteil und 1 bis 3 Kohlenstoffatomen im Alkylteil, gegebenenfalls substituiertes Alkoxy oder Alkylthio mit 1 bis 6 Kohlenstoffatomen, gegebenenfalls substituiertes Phenoxy gegebenenfalls substituiertes Benzyloxy, Amino, gegebenenfalls substituiertes Alkylamino oder Dialkylamino mit 1 bis 6 Kohlenstoffatomen je Alkylrest oder gegebenenfalls substituiertes Phenylamino steht,
$R^7$ für gegebenenfalls substituiertes geradkettiges oder verzweigtes Alkyl mit 1 bis 5 Kohlenstoffatomen, Phenyl oder Methylphenyl steht,
$R^8$ für Methoxycarbonyl, Trichlormethyl, Trifluormethyl oder Dichlorfluormethyl steht,
$R^2$, $R^3$, $R^4$ und $R^5$ gleich oder verschieden sein können und für Wasserstoff, Methyl, Ethyl, n- oder i-Propyl stehen, und
X für Wasserstoff, Halogen oder Nitro steht.

5. Zink-, Mangan- oder Kupfersalze von 1-Cyclopropyl-1,4-dihydro-4-oxo-3-chinolincarbonsäure-Derivaten der Formel (I) gemäß Anspruch 4, in der
$R^1$ für CO-$R^6$ CN, $SO_2$-$R^7$ oder S-$R^8$ steht, wobei
$R^6$ für Wasserstoff, für gegebenenfalls ein- bis dreifach durch Amino, Chlor, Alkoxycarbonyl mit 1 bis 4 Kohlenstoffatomen im Alkylteil, Carboxy, Alkoxy mit 1 bis 4 Kohlenstoffatomen, Hydroxy oder Trifluormethylthio substituiertes geradkettiges oder verzweigtes Alkyl mit 1 bis 5 Kohlenstoffatomen, gegebenenfalls ein- bis fünffach durch Fluor, Chlor, Hydroxy, Methoxy, Amino, Nitro oder Carboxy substituiertes Phenyl, für gegebenenfalls durch Amino im Methylenrest substituiertes Benzyl, gegebenenfalls durch Fluor, Chlor, Pyrazol-1-yl, 1,2,3-Triazol-1-yl, N-Oxido-2-, -3- oder -4-pyridyl substituiertes Alkoxy oder Alkylthio mit 1 bis 5 Kohlenstoffatomen, Benzyloxy, Amino, gegebenenfalls durch Alkoxycarbonyl mit 1 bis 3 Kohlenstoffatomen im Alkylteil, Benzyloxycarbonyl, Carboxy oder Halogen substituiertes Alkylamino mit 1 bis 5 Kohlenstoffatomen, für Phenylamino,
$R^7$ für gegebenenfalls ein- bis dreifach durch Fluor, Chlor oder Amino substituiertes geradkettiges oder verzweigtes Alkyl mit 1 bis Kohlenstoffatomen, für Phenyl oder Methylphenyl steht,
$R^8$ für Methoxycarbonyl, Trichlormethyl oder Dichlorfluormethyl steht, und
$R^2$, $R^3$, $R^4$ und $R^5$ Wasserstoff, Methyl oder Ethyl und
X Wasserstoff, Fluor, Chlor oder Nitro
bedeuten.

6. Zink-, Mangan- oder Kupfersalze von 1-Cyclopropyl-1,4-dihydro-4-oxo-3-chinolincarbonsäure-Derivaten der Formel (I) gemäß Anspruch 4, in der
$R^1$ für CO- $R^6$, CN, $SO_2$-$R^7$ oder S-$R^8$ steht,
$R^6$ für Wasserstoff, gegebenenfalls ein- oder dreifach durch Amino, Alkoxycarbonyl mit 1 bis 3 Kohlenstoffatomen im Alkylteil, Carboxy, Alkoxy mit 1 bis 3 Kohlenstoffatomen oder Trifluormethylthio

substituiertes geradkettiges oder verzweigtes Alkyl mit 1 bis 4 Kohlenstoffatomen, gegebenenfalls ein- bis fünffach durch Chlor, Hydroxy, Amino oder Carboxy substituiertes Phenyl, gegebenenfalls durch Amino substituiertes Benzyl, gegebenenfalle durch Pyrazol-1-yl, 1,2,3-Triazol-1-yl, N-Oxido-2-, -3- oder -4-pyridylmethyl substituiertes Alkoxy mit 1 bis 4 Kohlenstoffatomen, Alkylthio mit 1 oder 2 Kohlenstoffatomen, Benzyloxy, Amino, gegebnenfalls durch Alkoxycarbonyl mit 1 bis 3 Kohlenstoffatomen im Alkylteil oder Carboxy substituiertes Alkylamino mit 1 bis 5 Kohlenstoffatomen steht,

$R^7$ für geradkettiges oder verzweigtes Alkyl mit 1 bis 3 Kohlenstoffatomen, Dichlorfluormethyl, Chlorbutyl, Phenyl oder Methylphenyl steht,

$R^8$ für Methoxycarbonyl, Trichlormethyl oder Dichlorfluormethyl steht,

$R^2$ für Wasserstoff, Methyl oder Ethyl steht,

$R^3$ für Wasserstoff steht,

$R^4$ für Wasserstoff oder Methyl steht,

$R^5$ für Wasserstoff

und

X für Wasserstoff, Fluor, Chlor oder Nitro

stehen.

## Claims

1. Use of 1-cyclopropyl-1,4-dihydro-4-oxo-3-quinolinecarboxylic acid derivatives of the formula (I)

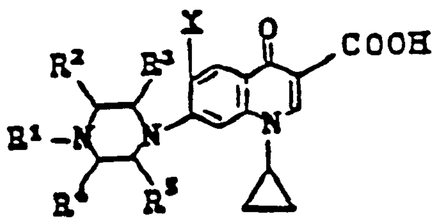

(I)

in which

$R^1$ represents a CO-$R^6$, CN, $SO_2$-$R^7$ or S-$R^8$ radical,

wherein

$R^6$ represents hydrogen, optionally substituted straight-chain or branched alkyl with 1 to 6 carbon atoms, optionally substituted aryl with 6 or 10 carbon atoms, optionally substituted aralkyl with 6 carbon atoms in the aryl part and 1 to 3 carbon atoms in the alkyl part, optionally substituted alkoxy or alkylthio with 1 to 6 carbon atoms, optionally substituted phenoxy, optionally substituted benzyloxy, amino, optionally substituted alkylamino or dialkylamino with 1 to 6 carbon atoms per alkyl radical or optionally substituted phenylamino,

$R^7$ represents optionally substituted straightchain or branched alkyl with 1 to 5 carbon atoms, phenyl or methylphenyl,

$R^8$ represents methoxycarbonyl, trichloromethyl, trifluoromethyl or dichlorofluoromethyl,

$R^2$, $R^3$, $R^4$ and $R^5$ can be identical or different and represent hydrogen, methyl, ethyl or n- or i-propyl and

X represents hydrogen, halogen or nitro, and their acid addition, alkali metal and alkaline earth metal salts, zinc, manganese and copper salts and hydrates, for combating bacteria in plant protection.

2. Use of the compounds of the formula (1) according to Claim 1,

in which

$R^1$ represents CO-$R^6$, CN, $SO_2$-$R^7$ or S-$R^8$, wherein

$R^6$ represents hydrogen, straight-chain or branched alkyl which has 1 to 5 carbon atoms and is optionally mono- to tri-substituted by amino, chlorine, alkoxycarbonyl with to 4 carbon atoms in the alkyl part, carboxyl, alkoxy with 1 to 4 carbon atoms, hydroxyl or trifluoromethylthio, phenyl whhich is optionally mono- to pentasubstituted by fluorine, chlorine, hydroxyl, methoxy, amino, nitro or carboxyl, benzyl which is optionally substituted by amino in the methylene radical, alkoxy or alkylthio which has 1 to 5 carbon atoms and is optionally substituted by fluorine, chlorine, pyrazol-1-yl, 1,2,3-triazol-1-yl or N-oxido-2-, 3- or -4-pyridyl, benzyloxy, amnino, alkylamnino which has 1 to 5 carbon atoms and is optionally substituted by alkoxycarbonyl with 1 to 5 carbon atoms in the alkyl part, benzyloxycarbonyl, carboxyl or halogen, or phenylamino,

$R^7$ represents straight-chain or branched alkyl which has 1 to 4 carbon atoms and is optionally mono- to tri-substituted by fluorine, chlorine or amino, or phenyl or methylphenyl,

$R^8$ represente methoxycarbonyl, trichloromethyl or dichlorofluoromethyl, and
$R^2$, $R^3$, $R^4$ and $R^5$ denote hydrogen, methyl or ethyl and
X denotes hydrogen, fluorine, chlorine or nitro, for combating bacteria in plant protection

3. Use compounds or the formula (1) accordingto Claim 1,
in which
$R^1$ represents CO-$R^6$, CN, $SO_2$-$R^7$ or S-$R^8$,
$R^6$ represents hydrogen, straight-chain or branched alkyl which has 1 to 4 carbon atoms and is optionally mono- or di-substituted by amino, alkoxycarbonyl with 1 to 3 carbon atoms in the alkyl part, carboxyl, alkoxy with 1 to 3 carbon atoms or trifluoromethylthio, phenyl which is optionally mono- to pentasubstituted by chlorine, hydroxyl, amino or carboxyl, benzyl which is optionally substituted by amino in the methylene radical, alkylthio with 1 or 2 carbon atoms or alkoxy with 1 to 4 carbon atoms which is optionally substituted by pyrazol-1-yl, 1,2,3-triazol-1-yl or N-oxido-2-, -3- or -4-pyridyl, benzyloxy, amino, or alkylamino which has 1 to 5 carbon atoms and is optionally substituted by alkoxycarbonyl with 1 to 3 carbon atoms in the alkyl part, chlorine or carboxyl,
$R^7$ represents straight-chain or branched alkyl with 1 to 3 carbon atoms, dichlorofluoromethyl, chlorobutyl, phenyl or methylphenyl,
$R^8$ represents methoxycarbonyl, trichloromethyl or dichlorofluoromethyl,
$R^2$ represents hydrogen, methyl or ethyl,
$R^3$ represents hydrogen,
$R^4$ represents hydrogen or methyl,
$R^5$ represents hydrogen and
X represents hydrogen, fluorine, chlorine or nitro, for combating bacteria in plant protection.

4. Zinc, manganese or copper salts of 1-cyclopropyl-1,4-dihydro-4-oxo-3-quinolinecarboxylic acid derivatives of the formula (I)

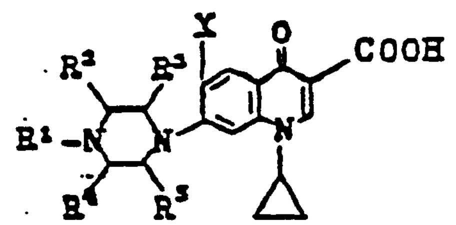

(I)

in which
$R^1$ represents a CO-$R^6$, CN, $SO_2$-$R^7$ or S-$R^8$ radical,
wherein
$R^6$ represents hydrogen, optionally substituted straight-chain or branched alkyl with 1 to 6 carbon atoms, optionally substituted aryl with 6 or 10 carbon atoms, optionally substituted aralkyl with 6 carbon atoms in the aryl part and 1 to 3 carbon atoms in the alkyl part, optionally substituted alkoxy or alkylthio with 1 to 6 carbon atoms, optionally substituted phenoxy, optionally substituted benzyloxy, amino, optionally substituted alkylamino or dialkylamino with 1 to 6 carbon atoms per alkyl radical or optionally substituted phenylamino,
$R^7$ represents optionally substituted straight-chain or branched alkyl with 1 to 5 carbon atoms, phenyl or methylphenyl,
$R^8$ represents methoxycarbonyl, trichloromethyl, trifluoromethyl or dichlorofluoromethyl,
$R^2$, $R^3$, $R^4$ and $R^5$ can be identical or different and represent hydrogen, methyl, ethyl or n- or i-propyl and
X represents hydrogen, halogen or nitro.

5. Zinc, manganese or copper salts of 1-cyclopropyl-1,4-dihydro-4-oxo-3-quinolinecarboxylic acid derivatives of the formula (I) according to Claim 4,
in which
$R^1$ represents CO-$R^6$, CN, $SO_2$-$R^7$ or S-$R^8$, wherein
$R^6$ represents hydrogen, straight-chain or branched alkyl which has 1 to 5 carbon atoms and is optionally mono- to tri-substituted by amino, chlorine, alkoxycarbonyl with 1 to 4 carbon atoms in the alkyl part, carboxyl, alkoxy with 1 to 4 carbon atoms, hydroxyl- or trifluoromethylthio, phenyl which is optionally mono- to pentasubstituted by fluorine, chlorine, hydroxyl, methoxy, amino, nitro or carboxyl, benzyl which is optionally substituted by amino in the methylene radical, alkoxy or alkylthio which has 1 to 5 carbon atoms and is optionally substituted by fluorine, chlorine, pyrazol-1-yl, 1,2,3-triazol-1-yl or N-oxido-2-, -3- or -4-pyridyl, benzyloxy, amino, alkylamino which has 1 to 5 carbon atoms and is optionally substituted by alkoxycarbonyl with 1 to 3 carbon atoms in the alkyl part, benzyloxycarbonyl, carboxyl or halogen, or phenylamino,

$R^7$ represents straight-chain or branched alkyl which has 1 to 4 carbon atoms and is optionally mono- to tri-substituted by fluorine, chlorine or amino, phenyl or methylphenyl,
$R^8$ represents methoxycarbonyl, trichloromethyl or dichlorofluoromethyl, and
$R^2$, $R^3$, $R^4$ and $R^5$ denote hydrogen, methyl or ethyl and
X denotes hydrogen, fluorine, chlorine or nitro.

6. Zinc, manganese or copper salts of 1-cyclopropyl-1,4-dihydro-4-oxo-3-quinolinecarboxylic acid derivatives of the formula (I) according to Claim 4,
in which
$R^1$ represents $CO-R^6$, CN, $SO_2-R^7$ or $S-R^8$,
$R^6$ represents hydrogen, straight-chain or branched alkyl which has 1 to 4 carbon atoms and is optionally mono- or tri-substituted by amino, alkoxycarbonyl with 1 to 3 carbon atoms in the alkyl part, carboxyl, alkoxy with 1 to 3 carbon atoms or trifluoromethylthio, phenyl which is optionally mono- to pentasubstituted by chlorine, hydroxyl, amino or carboxyl, benzyl which is optionally substituted by amino, alkoxy which has 1 to 4 carbon atoms and is optionally substituted by pyrazol-1-yl, 1,2,3-triazol-1-yl or N-oxido-2-, -3- or -4-pyridylmethyl, alkylthio with 1 or 2 carbon atoms, benzyloxy, amino, or alkylamino which has 1 to 5 carbon atoms and is optionally substituted by alkoxycarbonyl with 1 to 3 carbon atoms in the alkyl part or carboxyl,
$R^7$ represents straight-chain or branched alkyl with 1 to 3 carbon atoms, dichlorofluoromethyl, chlorobutyl, phenyl or methylphenyl,
$R^8$ represents methoxycarbonyl, trichloromethyl or dichlorofluoromethyl,
$R^2$ represents hydrogen, methyl or ethyl,
$R^3$ represents hydrogen,
$R^4$ represents hydrogen or methyl,
$R^5$ represents hydrogen and
X represents hydrogen, fluorine, chlorine or nitro.

**Revendications**

1. Utilisation de dérivés de l'acide-1-cyclopropyl-1,4-dihydro-4-oxo-3-quinoléine-carboxylique de formule I

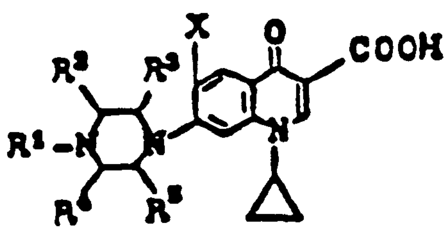

(I)

dans laquelle
$R^1$ représente un reste $CO-R^6$, CN, $SO_2-R^7$ ou $S-R^8$, dans lesquels $R^6$ représente l'hydrogène, un groupe alkyle à chaîne droite ou ramifiée en $C_1$-$C_6$ éventuellement substitué, un groupe aryle à 6 ou 10 atomes de carbone éventuellement substitué, un groupe aralkyle contenant 6 atomes de carbone dans la partie aryle et 1 à 3 atomes de carbone dans la partie alkyle, éventuellement substitué, un groupe alcoxy ou alkylthio en $C_1$-$C_6$ éventuellement substitué, un groupe phénoxy éventuellement substitué, un groupe benzyloxy éventuellement substitué, amino, alkylamino ou dialkylamino contenant 1 à 6 atomes de carbone dans chaque groupe alkyle, éventuellement substitué, ou phénylamino éventuellement substitué,
$R^7$ représente un groupe alkyle à chaîne droite ou ramifiée en $C_1$-$C_5$ éventuellement substitué, phényle ou méthylphényle,
$R^8$ représente un groupe méthoxycarbonyle, trichlorométhyle, trifluorométhyle ou dichlorofluorométhyle,
$R^2$, $R^3$, $R^4$ et $R^5$, ayant des significations identiques ou différentes, représentent chacun l'hydrogrène, un groupe méthyle, éthyle, n- ou iso-propyle, et
X représente l'hydrogène, un halogène ou un groupe nitro, et de leurs sels formés par addition avec des acides, sels alcalins et alcalino-terreux, sels de zinc, de manganèse et de cuivre et hydrates, pour la lutte contre les bactéries dans la protection des végétaux.

2. Utilisation des composés de formule I selon la revendication 1, dans laquelle

$R^1$ représente CO-$R^6$, CN, $SO_2$-$R^7$ ou S-$R^8$, dans lesquels

$R^6$ représente l'hydrogène, un groupe alkyle à chaîne droite ou ramifiée en $C_1$-$C_5$ portant éventuallemant un à trois substituants choisis parmi les groupes amino, le chlore, les groupes alcoxycarbonyle contenant 1 à 4 atomes de carbone dans la partie alkyle, carboxy, alcoxy en $C_1$-$C_4$, hydroxy ou trifluorométhylthio, un groupe phényle portant éventuellement un à cinq substituants choisis parmi le fluor, le chlore, les groupes hydroxy, méthoxy, amino, nitro ou carboxy, un groupe benzyle éventuellement substitué par un groupe amino dans le reste méthylène, un groupe alcoxy ou alkylthio en $C_1$-$C_5$ éventuellement substitué par le fluor, le chlore, un groupe pyrazole-1-yle, 1,2,3-triazole-1-yle, N-oxydo-2-, -3- ou -4-pyridyle, un groupe benzyloxy, amino, alkylamino en $C_1$-$C_5$ éventuellement substitué par un groupe alcoxycarbonyle contenant 1 à 3 atomes de carbone dans la partie alkyle, benzyloxycarbonyle, carboxy ou un halogène, ou un groupe phénylamino,

$R^7$ représente un groupe alkyle à chaîne droite ou ramifiée en $C_1$-$C_4$ portant éventuellement 1 à 3 substituants choisis parmi le fluor, le chlore et les groupes amino, un groupe phényle ou méthylphényle,

$R^8$ représente un groupe méthoxycarbonyle, trichlorométhyle ou dichlorofluorométhyle, et

$R^2$, $R^3$, $R^4$ et $R^5$ représentent l'hydrogène, des groupes méthyle ou éthyle, et

X représente l'hydrogène, le fluor, le chlore ou un groupe nitro, pour la lutte contre les bactéries dans la protection des végétaux.

3. Utilisation des composés de formule I selon la revendication 1, dans laquelle

$R^1$ représente CO-$R^6$, CN, $SO_2$-$R^7$ ou S-$R^8$,

$R^6$ représente l'hydrogéne, un groupe alkyle à chaîne droite ou ramifiée en $C_1$-$C_4$, portant le cas échéant un ou deux substituants choisis parmi les groupes amino, alcoxycarbonyle contenant 1 à 3 atomes de carbone dans la partie alkyle, carboxy, alcoxy en $C_1$-$C_3$ ou trifluorométhylthio, un groupe phényle portant éventuellement un à cinq substituants choisis parmi la chlore, les groupes hydroxy, amino ou carboxy, un groupe benzyle éventuellement substitué par un groupe amino dans le reste méthylène, un groupe alcoxy en $C_1$-$C_4$ ou alkylthio en $C_1$-$C_2$ éventuellement substitué par un groupe pyrazole-1-yle, 1,2,3-triazole-1-yle, N-oxydo-2-, -3- ou -4-pyridyle, un groupe benzyloxy, amino, alkylamino en $C_1$-$C_5$ éventuellement substitué par un groupe alcoxycarbonyle contenant 1 à 3 atomes de carbone dans la partie alkyle, le chlore ou un groupe carboxy.

$R^7$ represente un groupe alkyle a chaîne droite ou ramifiée en $C_1$-$C_3$, dichlorofluorométhyle, chlorobutyle, phényle ou méthylphényle,

$R^8$ représente un groupe méthoxycarbonyle, trichlorométhyle ou dichlorofluorométhyle,

$R^2$ représente l'hydrogène, un groupe méthyle ou éthyle,

$R^3$ représente l'hydrogène,

$R^4$ représente l'hydrogène ou un groupe méthyle,

$R^5$ représente l'hydrogène, et

X représente l'hydrogène, la fluor, la chlore ou un groupe nitro, pour la lutte contre les bactéries dans la protection des végétaux.

4. Sels de zinc, de manganèse ou de cuivre des dérivés de l'acide 1-cyclopropyle-1,4-dihydro-4-oxo-3-quinoléine-carboxylique de formule I

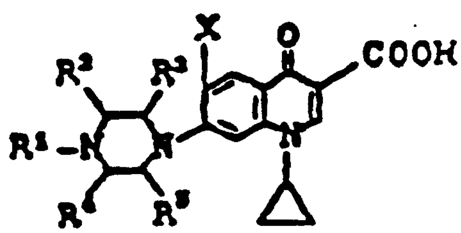

(I)

dans laquelle

$R^1$ représente un reste CO-R, CN, $SO_2$-$R^7$ ou S-$R^8$, dans lesquels

$R^6$ représente l'hydrogéne, un groupe alkyle à chaîne droite ou ramifiée en $C_1$-$C_6$ éventuellement substitué, un groupe aryle à 6 ou 10 atomes de carbone éventuellement substitué, un groupe aralkyle contenant 6 atomes de carbone dans la partie aryle et 1 à 3 atomes de carbone dans la partie alkyle, éventuellement substitué, un groupe alcoxy ou alkylthio en $C_1$-$C_6$ éventuellement substitué, un groupe phénoxy éventuellement substitué, un groupe benzyloxy éventuellement substitué, amino, alkylamino ou dialkylamino contenant 1 à 6 atomes de carbone dans chaque groupe alkyle, éventuellement substitué, ou phénylamino éventuellement substitué,

$R^7$ représente un groupe alkyle à chaîne droite ou ramifiée en $C_1$-$C_5$ éventuellement substitué, phényle ou méthylphényle,

$R^8$ représente un groupe méthoxycarbonyle, trichlorométhyle, trifluorométhyle ou dichlorofluorométhyle,

$R^2$, $R^3$, $R^4$ et $R^5$, ayant des significations identiques ou différentes, représentent chacun l'hydrogéne, un groupe méthyle, éthyle, n- ou iso-propyle, et

X représente l'hydrogène, un halogène ou un groupe nitro.

5. Sels de zinc, de manganèse ou de cuivre de dérivés de l'acide 1-cyclopropyl-1,4-dihydro-4-oxo-3-quinoléine-carboxylique de formule I selon la revendication 4, dans laquelle

$R^1$ représente CO-$R^6$, CN, $SO_2R$ ou S-$R^8$, dans lesquelles

$R^6$ représente l'hydrogène, un groupe alkyle à chaîne droite ou ramifiée en $C_1$-$C_5$ portant éventuellement un à trois substituants choisis parmi les groupes amino, le chlore, les groupes alcoxycarbonyle contenant 1 à 4 atomes de carbone dans la partie alkyle, carboxy, alcoxy en $C_1$-$C_4$, hydroxy ou trifluorométhylthio, phényle portant éventuellement un à cinq substituants choisis parmi le fluor, le chlore, des groupes hydroxy, méthoxy, amino, nitro ou carboxy, benzyle éventuellement substitué par un groupe amino dans le reste méthylène, alcoxy ou alkylthio en $C_1$-$C_5$ éventuellement substitué par le fluor, le chlore, un groupe pyrazole-1-yle, 1,2,3-triazole-1-yle, N-oxydo-2-, -3- ou -4-pyridyle, un groupe benzyloxy, amino, alkylamino en $C_1$-$C_5$ éventuellement substitué par un groupe alcoxycarbonyle contenant 1 à 3 atomes de carbone dans la partie alkyle, benzyloxycarbonyle, carboxy ou des halogènes, un groupe phénylamino,

$R^7$ représente un groupe alkyle à chaîne droite ou ramifiée en $C_1$-$C_4$ portant éventuellement un à trois substituants fluor, chlore ou amino, un groupe phényle ou méthylphényle,

$R^8$ représente un groupe méthoxycarbonyle, trichlorométhyle ou dichlorofluorométhyle et

$R^2$, $R^3$, $R^4$ et $R^5$ représentent l'hydrogène, des groupes méthyle ou éthyle, et

X représente l'hydrogène, le fluor, le chlore ou un groupe nitro.

6. Sels de zinc, de manganèse et de cuivre des dérivés de l'acide 1-cyclopropyl-1,4-dihydro-4-oxo-3-quinoléine-carboxylique de formule I selon la revendication 4, dans laquelle

$R^1$ représente CO-$R^6$, CN, $SO_2$-$R^7$ ou S-$R^8$,

$R^6$ représente l'hydrogène, un groupe alkyle à chaîne droite ou ramifiée en $C_1$-$C_4$ portant éventuellement un à trois substituants choisis parmi les groupes amino, alcoxycarbonyle contenant 1 à 3 atomes de carbone dans la partie alkyle, carboxy, alcoxy en $C_1$-$C_3$ ou trifluorométhylthio, un groupe phényle portant éventuellement un à cinq substituants choisis parmi le chlore, les groupes hydroxy, amino ou carboxy, un groupe benzyle éventuellement substitué par un groupe amino, un groupe alcoxy en $C_1$-$C_4$ éventuellement substitué par un groupe pyrazole-1-yle, 1,2,3-triazole-1-yle, N-oxydo-2-, -3- ou -4-pyridylméthyle, un groupe alkylthio en $C_1$-$C_2$, benzyloxy, amino, alkylamino en $C_1$-$C_5$ éventuellement substitué par un groupe alcoxycarbonyle contenant 1 à 3 atomes de carbone dans la partie alkyle ou par un groupe carboxy,

$R^7$ représente un groupe alkyle à chaîne droite ou ramifiée en $C_1$-$C_3$, dichlorofluorométhyle, chlorobutyle, phényle ou méthylphényle,

$R^8$ représente un groupe méthoxycarbonyle, trichlorométhyle ou dichlorofluorométhyle,

$R^2$ représente l'hydrogène, un groupe méthyle ou éthyle,

$R^3$ représente l'hydrogène,

$R^4$ représente l'hydrogène ou un groupe méthyle,

$R^5$ représente l'hydrogène, et

X représente l'hydrogène, le fluor, le chlore ou un groupe nitro.